# EUROPEAN PATENT SPECIFICATION

(11) **EP 2 158 482 B1**
(45) Date of publication and mention of the grant of the patent: **18.01.2017**
(21) Application number: 08826854.5
(22) Date of filing: 30.05.2008
(51) Int. Cl.: G01N 33/00, C12Q 1/68

(54) **HIGH SPECIFICITY AND HIGH SENSITIVITY DETECTION BASED ON STERIC HINDRANCE&ENZYME-RELATED SIGNAL AMPLIFICATION**
HOCHSPEZIFISCHE UND HOCHEMPFINDLICHE ERKENNUNG AUF BASIS VON DURCH STERISCHE HINDERUNG UND ENZYME VERMITTELTEN SIGNALVERSTÄRKUNGEN
DÉTECTION HAUTE SPÉCIFICITÉ ET HAUTE SENSIBILITÉ REPOSANT SUR UN EMPÊCHEMENT STÉRIQUE ET AMPLIFICATION DE SIGNAL ASSOCIÉE À UNE ENZYME

(30) Priority: 31.05.2007 US 941057 P
(43) Date of publication of application: 03.03.2010
(73) Proprietor: The Regents of the University of California, Oakland, CA 94607 (US)
(72) Inventor: WEI, Fang, Santa Monica, California 90402 (US); ZIMMERMANN, Bernhard G., San Mateo, California 94401 (US); WONG, David T., W., Beverly Hills, California 90211 (US)
(74) Representative: Campbell, Patrick John Henry
(86) International application number: PCT/US2008/065286
(87) International publication number: WO 2009/017878

(56) References cited:
- EP-A2- 0 745 690
- WO-A2-2007/033283
- US-A1- 2003 096 781
- US-A1- 2005 089 890
- US-A1- 2007 099 206
- WEI FANG ET AL: "Electrochemical detection of low-copy number salivary RNA based on specific signal amplification with a hairpin probe" NUCLEIC ACIDS RESEARCH, vol. 36, no. 11, June 2008 (2008-06), XP002581205 ISSN: 0305-1048
- MAO XUN ET AL: "Enzymatic amplification detection of DNA based on "molecular beacon" biosensors" BIOSENSORS & BIOELECTRONICS, vol. 23, no. 10, May 2008 (2008-05), pages 1555-1561, XP002581206 ISSN: 0956-5663
- BOCKISCH BENJAMIN ET AL: "Immobilized stem-loop structured probes as conformational switches for enzymatic detection of microbial 16S rRNA" NUCLEIC ACIDS RESEARCH, vol. 33, no. 11, 2005, XP002581207 ISSN: 0305-1048
- LIAO J C ET AL: "Use of electrochemical DNA biosensors for rapid molecular identification of uropathogens in clinical urine specimens" JOURNAL OF CLINICAL MICROBIOLOGY 200602 US LNKD- DOI:10.1128/JCM.44.2.561-570.2006, vol. 44, no. 2, February 2006 (2006-02), pages 561-570, XP002581208 ISSN: 0095-1137
- LI Y ET AL: "Salivary transcriptome diagnostics for oral cancer detection", CLINICAL CANCER RESEARCH, THE AMERICAN ASSOCIATION FOR CANCER RESEARCH, US, vol. 10, no. 24, 15 December 2004 (2004-12-15), pages 8442-8450, XP002448846, ISSN: 1078-0432, DOI: 10.1158/1078-0432.CCR-04-1167
- ANONYMOUS: 'MONOCLONAL ANTIBODIES AGAINST CYANINE 3 AND CYANINE 5 FLUOROPHORES', [Online] 10 April 2005, XP055244166 Retrieved from the Internet: <URL:https://www.funakoshi.co.jp/data/datas heet/KRA/MAB-002.pdf> [retrieved on 2016-01-22]
- TSOURKAS ANDREW ET AL: "Hybridization kinetics and thermodynamics of molecular beacons", NUCLEIC ACIDS RESEARCH, INFORMATION RETRIEVAL LTD, GB, vol. 31, no. 4, 15 February 2003 (2003-02-15), pages 1319-1330, XP002455592, ISSN: 0305-1048, DOI: 10.1093/NAR/GKG212

## Description

### CROSS-REFERENCES TO RELATED APPLICATIONS

This application claims priority to U.S. Provisional Patent Application No. 60/941,057, filed May 31, 2007.

### BACKGROUND OF THE INVENTION

### 1 FIELD OF THE INVENTION

The present invention relates to nucleic acid probes and assay methods.

### 2. DESCRIPTION OF THE RELATED ART

One of the requirements for point-of-care detection is to detect a small amount of a target molecule in a mixture. Detection of low number count target needs high sensitivity together with high specificity due to the complexity of any mixture. However, prior art detection methods require a compromise between specificity and sensitivity. Several techniques are developed to obtain the signal amplification, which helps to increase the sensitivity. In most detection methods, the signal intensity relates to the number of target within the detecting region, usually very small comparing to the whole sample volume. Hence either amplifying the amount of target in the whole sample volume or accumulating the target into a small detection region helps to high signal intensity.

The first method increases the total number of the target, probe and/or signal, thus brings out a high intensity measurement output. For example, PCR, Primed *in situ* labeling (PRINS) and nucleic acid sequence-based amplification (NASBA) technology are applied to increase the total amount of targets. See Monis and Giglio, In fection Genetics and Evolution, 2006. 6(1): p. 2-12. Ligase chain reaction (LCR) and rolling circle amplification (RCA) obtained the amplification of probes. Branched DNA (bDNA) and tyramide signal amplification (TSA) result in the signal amplification. See Andras et al., Molecular Biotechnology, 2001. 19(1): p. 29-44.

Compared to the direct amplification of target/probes/signal, the second method focuses on increasing the local concentration of target instead of creating more copies of the target. For example, nanotechnology can concentrate the few copies of the target within the sample into a detection region by applying nano-particle based techniques. Increase of both the whole amount and the local concentration of target results in high sensitivity. However, it would also produce a higher background level and more false-positive results since both the specific and non-specific signals would be amplified.

On the other hand, high specificity probes have been designed to decrease the background noise level, such as the molecular beacon and other probes having constraint structure. See Wei et al., Journal of the America Chemical Society, 2005. 127(15): p. 5306-5307; Broude, Trends in Biotechnology, 2002. 20(6): p. 249-256; Fan et al., Trends in Biotechnology, 2005. 23(4): p. 186-192; and Tyagi and Kramer, Nature Biotechnology, 1996. 14(3): p. 303-308. Typically, these methods are based on distance sensitive signal traducing process, such as FRET, intercalating dye (Howell et al., Genome Research, 2002. 12(9): p. 1401-1407) and electrochemistry. In these methods, binding of a specific target will cause the conformational change of the probes. The conformational change would result in dramatic switch into the signal ON state. However, by improving the specificity, those probes degrade the limit of detection because a large amount of target is required for a measurable signal, and hence introduce more false-negative results into the detection system.

Thus, a need still exists for assay methods and reagents that provide both high sensitivity and high specificity detection.

Bockisch et al. (2005) Nucleic Acids Research Vol. 33, No. 11, e101 discloses immobilized stem-loop structured probes as conformational switches for enzymatic detection of microbial 16S rRNA. Tsourkas et al. (2003) Nucleic Acids Research, Vol. 31, No. 4 1319-1330 describes the hybridization kinetics and thermodynamics of molecular beacons. EP-A-0745690 describes detectably labelled dual conformation oligonucleotide probes, assays and kits.

### BRIEF SUMMARY OF THE INVENTION

The present invention generally relates to probes and assay methods.

The invention provides a hairpin probe for detecting an mRNA or DNA biomarker comprising a polynucleotide sequence that specifically hybridizes to the sequence of the mRNA or DNA biomarker, and a ligand for a receptor wherein the receptor comprises a detectable label, said hairpin probe having a first three-dimensional structure in the absence of the mRNA or DNA biomarker being bound thereto and a second three-dimensional structure in the presence of the mRNA or DNA biomarker being bound thereto, wherein the first three-dimensional structure is a stem-loop structure where the stem is formed by the hybridization of two regions of the hairpin probe, wherein the first three-dimensional structure inhibits or prevents the receptor from binding the ligand and the second three-dimensional structure allows the receptor to specifically bind the ligand, and wherein one of the two regions of the stem and the loop of the hairpin probe is complementary to a part of the sequence of the mRNA or DNA biomarker, wherein the probe is immobilized on an electrochemical substrate, and wherein the first three-dimensional structure places the ligand at a position near the substrate such that the receptor is inhibited or prevented from binding the ligand.

In some embodiments, the detectable signal is amplified, such as by an enzymatic reaction. In some embodiments, the receptor is an antibody that specifically binds the ligand. In some embodiments, the detectable label is fluorescein, streptavidin, or biotin, and the like. In some embodiments, the detection signal from the detectable label may be amplified by way of enzymatic reactions catalyzed by peroxidase, laccase, glucose oxidase, alkaline phosphatease, or urease, and the like.

The invention also provides a method for detecting an mRNA or DNA biomarker in a sample, the method comprising:
contacting the probe as described above with the sample and the receptor, and
detecting the presence or absence of a complex between the ligand and the receptor,
wherein the presence of the complex indicates the presence of the mRNA or DNA biomarker in the sample and the absence of the complex indicates the absence of the mRNA or DNA biomarker in the sample.

In some embodiments, any receptor unbound to the ligand is removed prior to detecting the complex.

The invention provides a method to detect very low concentrations of a biomarker in a "dirty" sample (with high concentrations of contaminants and molecules that interfere with signal detection), such as saliva. It will have applications for situations where high quality sample preparation is not available or too expensive, such as point-of-care, and situations where biomarker concentration is very low compare to contaminants and inferring compounds (interferents). Feasibility data for low concentration detection is presented in an oral cancer mRNA biomarker, IL8.

In some embodiments, the probes are "ready-to-use," for instance, the probes are pre-anchored on surface and no other treatment during detection is required. In some embodiments, the probes are oligonucleotides or aptamers, which are biocompatible.

The probes of the present invention may be readily employed in multiplex applications and do not require expensive instruments and complicated data analysis.

The probes of the present invention reduce or eliminate false positive results over the prior art as prior art methods are not selective to the complementary and non-complementary targets, which results in false positive results. The steric hindrance effect to the probe design increases the specificity.

The probes of the present invention also reduce or eliminate false negative results over the prior art as prior art methods with high specificity results in signal decrease, which generates false negative results. Specific signal amplification was applied to increase the signal intensity thereby improving the sensitivity.

The probes and methods of the present invention may be used for clinical detection for biomarkers in blood, serum, urine and saliva samples, for example, salivary mRNA detection with original saliva and *in vivo* monitoring of the early stage of a disease.

The probes and methods of the present invention may be employed in multiplexed detection, *e.g*., a microarray comprising a plurality of probes according to the present invention, which are specific for different target nucleic acid molecules.

The probes of the present invention may be reused as the switch between different states of probes is reversible, such that the sensor is reusable. In some embodiments, the shape and size of the receptor and/or label bound thereto is optimized for the steric hindrance effect, *e.g*., a large receptor and/or label would be more sensitive to the steric hindrance than a relatively small receptor and/or label.

As disclosed herein, the present invention relates to a probe for a target nucleic acid molecule. The probe comprises a polynucleotide sequence (which specifically hybridizes to the sequence of the target nucleic acid molecule, *e.g*., IL-8 mRNA or DNA sequence) and a ligand for a receptor. This probe has a first three-dimensional structure in the absence of the target nucleic acid molecule being bound to the probe, and a second three-dimensional structure in the presence of the target nucleic acid being bound to the probe. The first three-dimensional structure inhibits or prevents the receptor from binding the ligand whereas the second three-dimensional structure allows the receptor to specifically bind the ligand.

The probe comprises a polynucleotide sequence that is complementary or substantially complementary to the target sequence. As used herein, "substantially complementary" refers to a sequence which specifically hybridizes to a sequence under moderate, preferably stringent, hybridization conditions. Some exemplary polynucleotide sequences are presented in Tables 2-4.

### BRIEF DESCRIPTION OF THE DRAWINGS

This invention is further understood by reference to the drawings wherein:
Figure 1 schematically illustrates an embodiment of the assay method of the present invention.
Figure 2 shows the influence of probe structure with hairpin probes according to the present invention.
Figure 3 shows the limit of detection for mRNA with linear probes and hairpin probes.
Figure 4 schematically illustrates the specific signal amplification in electrochemical detection with hairpin probe. When no target bound to hairpin probe, hairpin is closed and HRP cannot form an effective complex on the surface, therefore no signal is observed. After hybridized with target, the hairpin open up and the HRP complex is formed. Then TMB keeps regenerating the reduced HRP, which amplifies the current signal.
Figure 5 is a graph showing the cross-detection of with two sets salivary RNA applying hairpin probe: IL-8 and S100A8. The RNA target level is 5 nM for IL-8 and 7 nM for S100A8. The blank signal is 6xSSC and 10 mM MgCl₂. Mean and standard deviation of experiments performed 4 times are shown.
Figure 6 graphically compares detection with IL-8 hairpin probes with different linkers. The hairpin is closed in blank control and open up with RNA sample. The concentration of IL-8 RNA is 50 nM. The blank control is 6xSSC and 10 mM MgCl₂. Mean and standard deviation of experiments performed 4 times are shown. The configurations of the hairpin probes with different linker length are shown schematically.
Figure 7 graphically compares detection with IL-8 hairpin probes with different stem-loop structure. The hairpin is closed in blank control and open up with RNA sample. Sequences with underlines are complementary to the target RNA. Sequences in italic are the stem parts. Sequences in bold are the loop parts. HP1: 10 bp in stem and 41 bp in duplex. HP2: 8 bp in stem and 34 bp in duplex. HP3: 6 bp in stem and 27 bp in duplex. The concentration of IL-8 RNA is 50 nM. The blank control is 6xSSC and 10 mM MgCl₂. Mean and standard deviation of experiments performed 4 times are shown.
Figure 8 shows salivary RNA detection compared between linear and hairpin probe. (a): IL-8. Linear probes are IL-8 CP and IL-8 DP and hairpin probe is IL-8 HP as listed in Table 2; (b): S100A8. Linear probes are S100A8 CP and S100A8 DP and hairpin probe is S100A8 HP as listed in Table 2.
Figure 9 shows electrochemical detection of spiked saliva with IVT RNA. Circle: (a) IL8; (b) S100A8. The saliva sample is whole saliva from the same batch of the same person without any treatment.
Figure 10 depicts structures of linkages between the labeling molecules and oligonucleotides.
Figure 11 Cross-detection with two sets of IVT RNA applying HP: IL-8 and S100A8. (a) The amperometric signals for eight samples. (1)-(4) applied HPs for S100A8 and the targeting RNA were (1) 7 nM S100A8, (2) 500 nM IL-8, (3) 5 nM IL-8, and (4) buffer only, respectively. (5)-(8) used HPs for IL-8, and the targeting RNA were (5) 5 nM IL-8, (6) 700 nM S100A8, (7) 7 nM S100A8, and (8) buffer only, respectively. (b) Bar charts of the same eight samples in (a). The sequences for HPs are listed in Table 3 as IL-8 HP and S100A8 HP. Mean and standard deviation of four individual experiments are shown.
Figure 12 Salivary IL-8 RNA detection by the linear probe (LP) and HP. LPs were IL-8 CP and IL-8 DP, and HP was IL-8 HP as listed in Table 3. Blank control signals were subtracted from the measured signals. Mean and standard deviation of four experiments are shown. The data point of the 4 fM target for LP is not displayed, because its value was below that of the blank control.
Figure 13 Correlation between amperometric signals using HP and concentrations determined by qPCR of IL-8 mRNA for the same set of clinical saliva samples. The *R*² for linear regression was 0.99.

### DETAILED DESCRIPTION OF THE INVENTION

In previous detections related to conformational change (see Howell, W.M., M. Jobs, and A.J. Brookes, iFRET: an improved fluorescence system for DNA-melting analysis. Genome Research, 2002. 12(9): p. 1401-1407; Xiao, Y., et al., Single-step electronic detection of femtomolar DNA by target-induced strand displacement in an electrode-bound duplex. Proceedings of the National Academy of Sciences of the United States of America, 2006. 103(45): p. 16677-16680; and Xiao, Y., et al., Label-free electronic detection of thrombin in blood serum by using an aptamer-based sensor. Angewandte Chemie-International Edition, 2005. 44(34): p. 5456-5459), target recognition (specificity) and signal amplification (sensitivity) are two non-related steps. Only the recognition process is specific, while the amplification is non-specific and applies to both the signal and noise. In the present invention, amplification and recognition are both specific. Only the specific binding of target will cause signal amplification. Non-specific binding of other interferents (contaminants and other molecules in the sample to be assayed) contribute significantly less to the measured signal. Therefore, noise level is suppressed and only the target signal is amplified.

As used herein, "target" is used interchangeably with "target nucleic acid molecule". As used herein, a "target" nucleic acid molecule may be any nucleic acid molecule, the presence and/or amount of which is desired to be known. In some embodiments, the sequence of the target nucleic acid molecule is known. In some embodiments, e.g., mutation detection, the sequence of the target nucleic acid molecule may be a sequence that is suspected of having alterations, *i.e.* differences, from a reference nucleic acid sequence. In these embodiments, the sequence of the target nucleic acid molecule may or may not be known, and the "reference nucleic acid sequence" is a known nucleic acid sequence to which the sequence of the target nucleic acid molecule may be compared. The alteration in the target nucleic acid molecule may be in a single nucleotide base or more than a single nucleotide base. Such an alteration may be a known polymorphic alteration, such as a single nucleotide polymorphism.

As used herein, "nucleic acid molecule", "polynucleotide", and "oligonucleotide" are used interchangeably to refer DNA and RNA molecules of natural or synthetic origin which may be single-stranded or double-stranded, and represent the sense or antisense strand. The nucleic acid molecules may contain known nucleotide analogs or modified backbone residues or linkages, and any substrate that can be incorporated into a polymer by DNA or RNA polymerase. Examples of such analogs inlude phospborothioates, phosphoramidates, methyl phosphonates, chiral-methyl phosphonates, 2-O-methyl ribonucleotides, peptide-nucleic acids (PNAs), and the like.

Preferably, the nucleic acid molecule is isolated. As used herein, "isolated" refers to a nucleic acid molecule that is isolated from its native environment. An "isolated" nucleic acid molecule may be substantially isolated or purified from the genomic DNA of the species from which the nucleic acid molecule was obtained. An "isolated" polynucleotide may include a nucleic acid molecule that is separated from other DNA segments with which the nucleic acid molecule is normally or natively associated with at the 5' end, 3' end, or both.

The nucleic acid molecules may be readily prepared by methods known in the art, for example, directly synthesizing the nucleic acid sequence using methods and equipment known in the art such as automated oligonucleotide synthesizers, PCR technology, recombinant DNA techniques, and the like.

The nucleic acid molecules may contain a label. A wide variety of labels and conjugation techniques are known by those skilled in the art and may be used in various nucleic acid and amino acid assays employing the nucleic acid molecules described.

As used herein a "label" or a "detectable label" is a composition or molecule that produces a signal detectable by methods known in the art including radiography, fluorescence, chemiluminescence, enzymatic activity, absorbance, and the like. Detectable labels include radioisotopes, fluorophores, chromophores, enzymes, dyes, metal ions, ligands such as biotin, avidin, strepavidin and haptens, quantum dots, and the like.

A "labeled " nucleic acid molecule comprises a bound label such that the presence of the nucleic acid molecule may be detected by detecting the presence of the label bound to thereto. The label may be bound to the nucleic acid molecule via a covalent bond, such as a chemical bond, or a noncovalent bond, such as ionic, van der Waals, electrostatic, or hydrogen bonds. Methods known in the art for producing labeled hybridization or PCR probes for detecting sequences related to polynucleotides may be used and include oligolabeling, nick translation, end-labeling or PCR amplification using a labeled nucleotide, and the like, preferably end-labeling. Suitable labels that may be used include radionucleotides, enzymes, fluorescent, chemiluminescent, or chromogenic agents as well as substrates, cofactors, inhibitors, magnetic particles, and the like.

As used herein, a "nucleic acid probe" or "probe" refers to a nucleic acid molecule that is capable of binding to a target nucleic acid molecule having a sequence that is complementary to the sequence of the nucleic acid probe. A probe may include natural or modified bases known in the art. *See e.g.* MPEP 2422, 8th ed. The nucleotide bases of the probe may be joined by a linkage other than a phosphodiester bond, so long as the linkage does not interfere with the ability of the nucleic acid molecule to bind a complementary nucleic acid molecule. The probe may bind a target sequence that is less than 100% complementary to the probe sequence and such binding depends upon the stringency of the hybridization conditions. The presence or absence of the probe may be detected to determine the presence or absence of a target sequence or subsequence in a sample. The probe may contain a detectable label.

As used herein, "assaying" is used interchangeably with "detecting," "measuring," "monitoring," and "analyzing."

As used herein, "affixed," "attached," "associated," "conjugated," "connected," "coupled," "immobilized," "adsorbed," and "linked" are used interchangeably and encompass direct as well as indirect connection, attachment, linkage, or conjugation, which may be reversible or irreversible, unless the context clearly dictates otherwise.

As provided herein, a "ligand" refers to a molecule that binds to another molecule, *i.e*., a "receptor." For example, an antigen binding to an antibody, oligonucleotides that hybridize to complimentary oligonucleotides, a hormone or neurotransmitter binding to a receptor, or a substrate or allosteric effector binding to an enzyme and include natural and synthetic biomolecules, such as proteins, polypeptides, peptides, nucleic acid molecules, carbohydrates, sugars, lipids, lipoproteins, small molecules, natural and synthetic organic and inorganic materials, synthetic polymers, and the like. As provided herein, a "receptor" is a molecule that specifically binds a given ligand.

As used herein, "specific binding" or "specific interaction" between two molecules means that a given ligand and its receptor bind or interact with each other with specificity sufficient to differentiate from the binding of or interaction with other components or contaminants in a given sample.

As used herein, the phrase "selectively (or specifically) hybridizes to" refers to the binding, duplexing, or hybridizing of a nucleic acid molecule to a particular nucleotide sequence over other nucleotide sequences under stringent hybridization to moderate hybridization conditions. For selective or specific hybridization, a positive signal is at least about 2 times, preferably about 5 times, more preferably about 10 times the background hybridization. Stringent hybridization conditions are about 5 °C below the thermal melting temperature (*T*ₘ) of the probe to about 10 °C below *T*ₘ. Moderate hybridization conditions are about 10 °C below the thermal melting temperature (*T*ₘ) of the probe to about 20 °C to about 25 °C below *T*ₘ.

High sensitivity: In order to increase the sensitivity, signal amplification based on sandwich detection is applied. The fundamental concept of sandwich amplification is the application of a mediator to form sandwich-like complex, with a purpose of amplifying the signal. First, after target binds to the probe, a complex forms between reporter labeled probe and mediator before detection. Then the excess mediator is removed and detection is carried out. See Liao, J.C., et al., Use of electrochemical DNA biosensors for rapid molecular identification of uropathogens in clinical urine specimens. Journal of Clinical Microbiology, 2006. 44(2): p. 561-570; and Gau, V., et al., Electrochemical molecular analysis without nucleic acid amplification. Methods, 2005. 37(1): p. 73-83. In conventional sandwich detection of nucleic acids, the oligonucleotide probes are linear. Therefore both the non-specific and specific target, independent of any mediator binding, would increase background and cause false-positive results.

High specificity: In order to increase the specificity, a steric hindrance-switch structure (such as a stem-loop and aptamer) is introduced to the probe design. The probes of the present invention have at least a two-state structure. When no target is bound, the probe stays in structure I. In the structure I state, reporters (alternatively referred to as "ligands") cannot form effective complex with a mediator (alternatively referred to as a "receptor" which specifically binds a given ligand) because of the receptor is sterically hindered, inhibited or prevented from coming into contact with the ligand. After binding with a target, the probe turns into structure II. In the structure II state, a reporter forms an effective complex with the mediator which results in a signal amplification. The steric hindrance design is simple and effective, without any additional chemical reaction step that would increase labor and cost.

A physical force parameter *F*ₐ describes the intra-molecular interaction of the probe that constraints conformation. Higher *F*ₐ makes the probe more stable in the structure I state. Another physical force parameter *F*_{b} describes the inter-molecular interaction between target and probe. Higher *F*_{b} enables the probe to stabilize in the structure II state. Competition between *F*ₐ and *F*_{b} determines which state the probe stays in. Since *F*_{b} comes from the interaction between target and probe, a specific target binding will produce a higher *F*_{b} than a non-specific target binding. Therefore, the specificity of the detection can be determined by the difference between |*F*ₐ-*F*_{b}(specific)| and |*F*ₐ-*F*_{b}(non-specific)|. In most cases, the target, and therefore the interaction between target and probe, *F*_{b}, cannot be changed. In order to achieve high specificity, however, one can design the *F*ₐ to desired value via changing the probe design.

Low copy-number application: Furthermore, comparing to the traditional conformational-based detection which are usually signal-off processes (Fan, C.H., K.W. Plaxco, and A.J. Heeger, Electrochemical interrogation of conformational changes as a reagentless method for the sequence-specific detection of DNA. Proceedings of the National Academy of Sciences of the United States of America, 2003. 100(16): p. 9134-9137), detection according to the present invention may be a signal-on process. Signal-on process detects an increase of the signal in a low background value, while signal-off process detects a decrease of the signal a high background value. Usually a measurement at high value has a larger error than that of lower value, so a signal-on process has a more steady background noise level. Furthermore, the dynamic range for the decrease of the signal is limited by the original background value in the signal-off process. Therefore, the signal-on process has a higher limit of detection, less measurement error, as well as more convenient for commercial use because it has less signal processing steps than signal-off process.

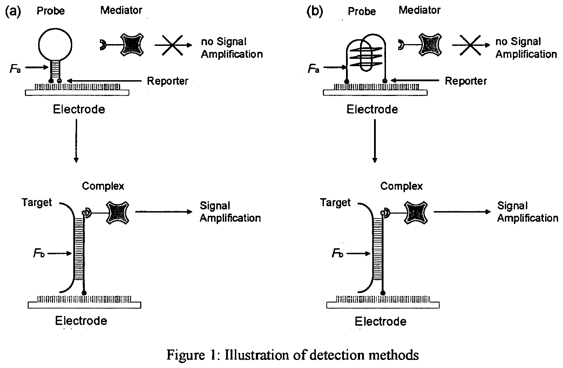

Probe design: The bio-recognition part and constraint-structure (or steric-switch) part of the probe can be designed separately or integrated. Figure 1 illustrates the two methods of probe design. In the separately-design method (Figure 1A), a DNA hairpin structure was used as the probe. The loop is the bio-recognition part for the target. The stem is designed for steric-switch part. When the specific target concentration is below limit of detection, the probe remains as a closed structure, creating a conformational restriction that prevents the reporter from forming an effective complex with the mediator. Hence the measured signal level is low. After a binding with a specific target, the hairpin opens and the reporter is free to from the effective complex with the mediator that amplifies the signal, hence the measure signal level is high. In the integrated design, the composition of the probe can form a constraint structure itself without additional part required in the probe design. Here a G-quadruplex may be used for instance (Figure 1B).

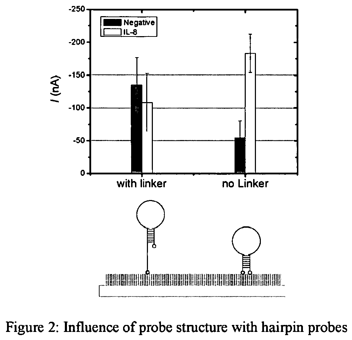

Figure 2 shows the effects of different levels of designed steric hindrance. Here two hairpin probes with a linker (located between the probe and the substrate) and without the linker, each having a different level of steric hindrance due to the reporters' proximity to the electrode surface to which the probes are bonded, were compared. In this setup, hybridization with specific target forms a DNA duplex that separates the report further away from the electrode surface, and hence a decrease in signal output is measured. Note that in this signal-off process, the decrease of the high background value due to this signal is small and is difficult to detect. For the probe with the linker (a lower designed steric hindrance), even when the hairpin is closed, the reporter is far away from the surface such that the complex between report and mediator can still form and is effective. Therefore the measured output between no target and existence of target (IL-8) is small, as shown in the left data set labeled "with linker." When the steric hindrance is designed to be greater by removing the linker from the hairpin probe, the reporter is very close to surface when no target is bonded, preventing the formation of an effective mediator-reporter complex and therefore very low background noise is measured. After hybridization with target, the distance between reporter and surface increases and the complex is allowed to form and effectively amplifies the signal. The change of the signal is dramatic with a large signal to noise ratio. This is a signal-on process and the result is shown on the right data set labeled "no-linker."

Although the probes exemplified herein are on the surface of a substrate, the detection may be carried out using the probes in solution. Since the key innovation of the probe design is the steric-hindrance, all types of constraints that cause the steric-hindrance could be applied. For instance, the probes may bond to the nanoparticles, magnetic beads, or macromolecules such as proteins.

Signal read out: The read-out signal is electrical output. If the amplification is related to electron transfer process, the signal is preferably current/voltage.

Since the signal read-out of the present invention is related to the complex formed between reporter and mediator, the target for detection may be label-free. Label-free detection not only decreases the cost of reagent use, but also makes it possible for real time and high throughput detection. It can be applied to micro-array and *automatic in situ* detection.

### EXAMPLES

The following examples are provided by way of illustration only and not by way of limitation. Those of skill in the art will readily recognize a variety of non-critical parameters that could be changed or modified to yield essentially the same or similar results.

### Example 1: Monitoring low concentration of mRNA using the hairpin probe design

mRNA biomarkers in saliva show that saliva can act as a diagnostic fluid for the oral disease, and possibly for other systematic disease. However, concentration of specific mRNA biomarker in saliva is below femto mol/L. In addition, large excess of non-specific mRNA, rRNA and protein coexist. The key point is how to detect tiny amounts of mRNA or protein in saliva without purification and amplification.

Disclosed herein includes an electrochemical array to IL8, an mRNA biomarker for oral cancer. Use of a probe according to the present invention is exemplified. The probe is designed as hairpin structure. The reporter is the detection probe (fluorescein-green). The mediator is the anti-fluorescein-HRP conjugate. The signal amplification is based on HRP redox process. The signal read-out is current. *F*ₐ is the Gibbs free energy of hairpin probe. *F*_{b} is the Gibbs free energy of duplex formed between probe and target.

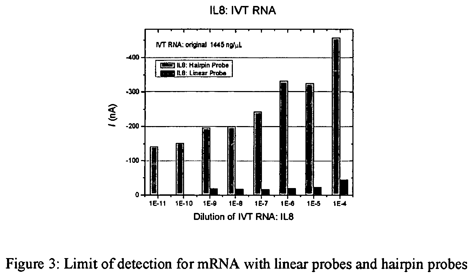

By applying hairpin probes without linker, the limit of detection (LOD) of IL8 is about 10 fg/mL (about 1 fmol/L) (Figure 3). For the linear probes, the LOD is only about 100 pg/mL (10 pmol/L). The dynamic range for hairpin probe detection is from 10 fg/mL to 100 ng/mL.

Saliva, as a mirror of the body fluid, has been proved to reflect the normal and disease states of the body. See, *e.g*., I. D. Mandel, J. Am. Dent. Assoc., 124:85-87 (1993); I. D. Mandel, J. Oral Pathol. Med., 19:119-125 (1990); D. T. Wong, J. Am. Dent. Assoc., 137:313-321 (2006). Recently, Wong's group has observed several salivary mRNAs were consistently elevated in saliva from oral cancer patients. See D. T. Wong, J. Am. Dent. Assoc., 137:313-321 (2006). Among these mRNA, four in combination (OAZ-1, SAT, IL8 and IL1-β) can serve as biomarkers to discriminate saliva of oral cancer patients from that of control subjects. See Y. Li et al., Clin. Cancer Res., 10:8442-8450 (2004). The identification of mRNA biomarker makes saliva a valuable diagnostic fluid. See S. Hahn et al., Bioelectrochemistry, 67:151-154 (2005). To date, however, there is no consistent and reliable technology for direct RNA detection in unextracted saliva. Comparing to other fluid-based detection, such as blood and urine, saliva-based diagnostics is more accessible, accurate, and inexpensive than current methodologies while presenting less risk for the patient.

A major concern of saliva as a diagnostic fluid is that the biomarkers are generally present in lower amounts in saliva than in serum. Due to the low concentration of salivary biomarkers and the complexity of saliva, conventional detection methods cannot meet the clinical diagnostic requirement for high signal-to-noise ratio.

Several techniques are developed to obtain signal amplification, which helps to increase the sensitivity. In most detection methods, the signal intensity relates to the number of targets within the detecting region, usually very small, compared with the whole sample volume. Hence either amplifying the amount of target in the whole sample volume or accumulating the target into a small detection region are applied to ensure high signal intensity. The first method increases the total number of the target, probe and/or signal, thus generates a high intensity measurement output. For example, PCR, *primed in situ* labeling (PRINS) and nucleic acid sequence-based amplification (NASBA) technology are applied to increase the total amount of targets. See P. T. Monis and S. Giglio, Infect. Genet. Evol., 6:2-12 (2006). Ligase chain reaction (LCR) and a rolling circle amplification (RCA) amplify the probes. See P. T. Monis and S. Giglio, Infect. Genet. Evol., 6:2-12 (2006. Branched DNA (bDNA) and tyramide signal amplification (TSA) result in the signal amplification. See S. C. Andras et al., Mol. Biotechnol., 19:29-44 (2001). Compared with the direct amplification of target/probes/signal, the second method focuses on increasing the local concentration of target instead of creating more copies of the target. For example, nanotechnology can concentrate the few copies of the target within the sample into a detection region by applying nano-particle based techniques. See A. N. Shipway and I. Willner, Chem. Commun., pp. 2035-2045 (2001); A. Merkoci, Febs J., 274:310-316 (2007); J. Wang, Anal. Chim. Acta, 500:247-257 (2003); S. G. Penn et al., Curr. Opin. Chem. Biol., 7:609-615 (2003). Increase of both the whole amount and the local concentration of target results in high sensitivity. However, it would also produce a higher background level and more false-positive results since both the specific and non-specific signals would be amplified.

On the other hand, competition-based detections have been designed to decrease the background noise level. The detecting probes always have several quasi-stable states, where each state exhibits different level of signal intensity. See N. L. Goddard et al., Phys. Rev. Lett., 85:2400-2403 (2000); C. H. Fan et al., P Natl Acad Sci USA, 100:9134-9137 (2003); S. Tyagi and F. R. Kramer, Nat. Biotechnol., 14:303-308 (1996); F. Wei et al., Biosens. Bioelectron., 18:1149-1155 (2003); F. Wei et al., J. Am. Chem. Soc., 127:5306-5307 (2005). Competition between the complementary target and non-complementary target switches the probes between these states, thus presented as different level of signal. The switching process can be achieved by either intra-molecular or inter-molecular competition. For intra-molecular switch, usually the high specific probes are applied, which has 2 or more quasi-stable conformations, such as the molecular beacon and other probes having constraint structure. See C. H. Fan et al., P Natl Acad Sci USA, 100:9134-9137 (2003), S. Tyagi and F. R. Kramer, Nat. Biotechnol., 14:303-308 (1996), F. Wei et al., J. Am. Chem. Soc., 127:5306-5307 (2005); Y. Xiao et al., P Natl Acad Sci USA, 103:16677-16680 (2006); A. A. Lubin et al., Anal. Chem., 78:5671-5677 (2006); N. E. Broude, Trends Biotechnol., 20:249-256 (2002). Binding to a specific target will cause the conformational change of the probes. Typically, the conformational change will affect the signal level which is distance sensitive, such as FRET, intercalating dye and electrochemistry. See T. J. Huang et al., Nucleic Acids Research, vol. 30 (2002); V. Gau et al., Methods, 37:73-83 (2005). Non-specific targets don't generate signal change and the background level is low. However, by improving the specificity, those probes reduce the limit of detection, because a large amount of target is required for a measurable signal, and hence introduce more false-negative results into the detection system..

Regarding previous detections related to conformational change target recognition (specificity) and signal amplification (sensitivity) are two non-related steps. See C. H. Fan et al., P Natl Acad Sci USA, 100:9134-9137 (2003); S. Tyagi and F. R. Kramer, Nat. Biotechnol., 14:303-308 (1996); F. Wei et al., J. Am. Chem. Soc., 127:5306-5307 (2005). As disclosed herein, high sensitivity and high specificity are simultaneously achieved by a novel hairpin probe design that enables selective amplification. This hairpin probe comprises of a bio-recognition component highly specific to the target, integrated together with a constraint-structure component that activates the signal reporting process. Only after the bio-recognition component verifies the specificity of the target, would the constraint-structure component remove the built-in steric hindrance, permitting signal amplification to occur, which results in high sensitivity. When there is no specific target binding, the steric hindrance inhibits the signal amplification. Therefore, only with the specific target, even if present in a low copy number in a mixture with large amount of interferents, can generate a measurable signal. This method of selective amplification greatly suppresses non-specific bindings and the background level, overcoming the two key hurdles in the implementation of any point-of-care detection system.

### Materials

All the reagents (Table 1) are used as purchased or diluted with buffer solution without any pretreatment.

**Table 1 Materials for electrochemical RNA detection**

| Name | Company |
|---|---|
| 1-ethyl-3-(3-dimethylaminopropyl) carbodiimide hydrochloride (EDC) | Biacore |
| N-hydroxysuccinimide (NHS) | Biacore |
| Amine-PEO₂-Biotin Labeling Reagent (Ez-Biotin) | Pierce |
| Ethanolamine-HCl, 1.0 M, pH 8.5 | Biacore |
| Magnesium chloride (MgCl₂) | Sigma |
| Streptavidin | VWR |
| Anti-Fluorescein-HRP | Roche |
| 3,3',5,5' tetramethylbenzidine substrate low activity (TMB/H₂O₂) | Neogen |
| 1 × Phosphate-Buffered Saline liquid, contains no calcium nor magnesium | Invitrogen |
| 1 × Tris-HCL buffer (Tris-HCl) | Invitrogen |
| 20 × sodium citric acid buffer (SSC) | Invitrogen |
| Blocker BSA in PBS solution contains 10% bovine serum albumin, pH 7.4 | Pierce |
| Blocker Casein in PBS solution contains 1% (w/v) casein in PBS, pH 7.4 | Pierce |

The electrochemical sensor used was a 16-units gold array. See V. Gau et al., Methods, 37:73-83 (2005). Array of electrodes allows multiplexing detection of different samples simultaneously. For each unit, there are three-electrode setup including working electrode (WE), counter electrode (CE) and reference electrode (RE). As advantages of a tiny electrode array, the signal read-out of the 16 electrodes can be obtained simultaneously and only 4 µL of sample solution is needed for the detection. As exemplified herein, the electrochemical signal is the current generated by the redox process of reporting enzyme - horseradish peroxidase (HRP). After the reaction between HRP and H₂O₂, HRP turns into the oxidized state. TMB then keeps regenerating the reduced HRP via 2-electron step which amplifying the current signal.

### Detection protocols

The surface modification of the gold electrochemical sensor contains 3 steps as the following (V. Gau et al., Methods, 37:73-83 (2005); J. J. Gau et al., Biosens. Bioelectron., 16:745-755 (2001):

*Probes immobilization*: The gold electrodes were pre-coated with self-assembled monolayer which is terminated by carboxyl group. The gold surface was activated by 4µL 50% EDC and 50% NHS for 10 mins. After that the sensors are rinsed with DI water (18.3 MΩ·cm) and dried with ultra pure nitrogen gas. Then 4µL of 5mg/mL Ez-Biotin was loaded to the gold surface, followed by DI water rinse and dried with nitrogen gas. After that, 0.5 mg/mL streptavidin in PBS buffer containing 2.5% BSA was incubated on the electrode for 10 mins, finally achieving streptavidin coated electrodes. Then 4 µL of biotinylated and fluorescein dual-labeled hairpin probes (HP) in Tris-HCl buffer were immobilized onto the electrodes for 30 mins via the interactions between streptavidin on the surfaces and the biotin label on the probes. The excessive HPs were removed by a thorough rinse with DI water and dried with nitrogen gas.

*Targets Hybridization*: The surface was incubated with the target sample which is prepared in 6xSSC buffer containing 10 mM MgCl₂ for 60 mins. After the hybridization, the electrodes were rinsed with DI water and dried with nitrogen gas.

*Signal read-out*: The current is proportional to the surface concentration of the target. See V. Gau et al., Methods, 37:73-83 (2005). First, 0.5 mU/mL anti-fluorescein-HRP in 0.5% casein blocker solution was bound with the fluorescein labels on HP. After rinsed with DI water and dried with nitrogen gas, TMB/H₂O₂ substrate was added. Amperometry detection was carried out by applying -200 mV voltage to each electrode unit, followed by parallel signal read-out after 60s equilibrium.

The electrochemical signal is the current generated by the redox process of reporting enzyme - horseradish peroxidase (HRP). After the reaction between HRP and H₂O₂, HRP turns into the oxidized state. TMB then keeps regenerating the reduced HRP via 2-electron step which amplifying the current signal. First, 0.5 mU/mL anti-fluorescein-HRP in 0.5% casein blocker solution was bound with the fluorescein labels on HP. After rinsed with DI water, TMB/H₂O₂ was added. Amperometry detection was carried out by applying - 200 mV voltage to each electrode unit, followed by simultaneous signal read-out after 60s equilibrium. The current is proportional to the surface concentration of the target. See V. Gau et al., Methods, 37:73-83 (2005).

### Oligonucleotides probes and RNA sample preparation

Oligonucleotides were ordered from Operon (Alabama, USA). Every hairpin probes are labeled with biotin on one end and biotinTEG on the other. The biotin label can bind streptavidin as an anchor, and fluorescein label is a signal reporter respectively. Biotin-TEG provided by Operon has an extra 16-atom spacer connecting biotin and oligo chain. This spacing design confers to the biotin a good accessibility towards the streptavidin.

Two mRNA targets were selected for the detection. Interleukin 8 (IL8) is a salivary biomarker for oral cancer. The concentration of IL8 is 2 fM for healthy people and increased to about 16 fM in oral cancer sample. See Y. Li et al., Clin. Cancer Res., 10:8442-8450 (2004). In order to normalize the RNA level in the saliva sample, a reference gene, S100 calcium-binding protein A8 (S100A8), which shows no oral cancer relevance, was used. For saliva detection, S100A8 is used as a reference control on each electrochemical sensor.

In vitro transcript (IVT) RNA are prepared according to methods known in the art. See H. Ohyama et al., Biotechniques, 29:530-+ (2000). In brief, reverse transcription with T7-oligo-(dT)24 as the primer was performed to synthesize the first strand of c-DNA. The in vitro transcription was carried out with T7 RNA polymerase (Ambion Inc., Austin, TX, USA). 1 µL cellular RNA was reversely transcripted to make cDNA and 1 µl of cDNA used as template for PCR use primer with T7 sequence. The quantity and quality of cRNA were determined by spectrometry (NanoDrop Tech., Delaware, USA). The IVT RNA sample was aliquot and stored at -20°C before use. For assessment of sensitivity and specificity of saliva, the IVT RNA was spiked into saliva.

### Results and discussion

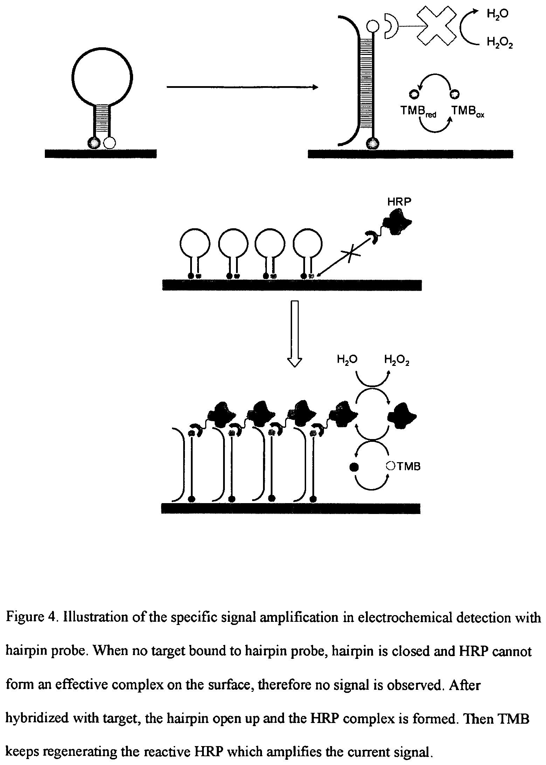

In conventional sandwich detection of nucleic acid, the oligonucleotide probes are linear. Therefore both the non-specific and specific target, independent of any mediator binding, would increase background and cause false-positive results. In order to increase the specificity, the steric hindrance effect was introduced into the hairpin structure. See Figure 4. The hairpin probe is characteristic of its open-or-not two-state structure. When no target is bound, the hairpin probe stays in closed state thus the reporters cannot form effective complex with mediator because of the designed steric hindrance. After binding with a specific target, the probe turns into open state and a reporter forms an effective complex with the mediator, resulting a signal amplification. The steric hindrance design is simple and effective, without any additional chemical reaction step that would increase efforts in carrying out the experiments. Since the signal read-out in this work is only related to the complex formed between reporter and mediator, the target for detection is label-free. Label-free detection not only decreases the types of reagent use, but also makes it possible for real time and high throughput detection. It can be applied to micro-array and *automatic in situ* detection.

The probes according to the present invention are based on surface steric hindrance which inhibiting the HRP/TMB signal amplification. Therefore, the distance between the surface and reporter would be the major factor to the recognition process. In this setup, hybridization with specific target forms a DNA duplex that separates the report further away from the electrode surface, and hence a decrease in signal output is measured. As the reporter moving from the surface into the solution, the surface restriction could diminished thus the current signal will increase.

Furthermore, comparing to the traditional conformational-based detection which are usually signal-off processes, the detection according to the present invention may be a signal-on process. Signal-on process detects an increase of the signal in a low background value, while signal-off process detects a decrease of the signal a high background value. Usually a measurement at high value has a larger error than that of lower value, so a signal-on process has a more steady background noise level. Furthermore, the dynamic range for the decrease of the signal is limited by the original background value in the signal-off process. Therefore, the signal-on process has a higher limit of detection, less measurement error, as well as more convenient for commercial use because it has less signal processing steps than signal-off process.

### Concept of steric hindrance effects with hairpin probe

The specific signal amplification is accomplished via combination of sandwich-like signal amplification by HRP and TMB/H₂O₂ and signal selectivity by hairpin probe design. The basic idea of the method of the present invention is the steric hindrance by surface which inhibits the HRP/TMB binding to target-free probe. Therefore, the distance between the surface and reporter would be a major factor to the recognition process. As the reporter moving from the surface into the solution, the surface restriction is diminished thus the current signal will increase. Without steric hindrance, hybridization with specific target forms a DNA duplex that separates the reporters from the electrode surface, and hence a decrease in signal output is measured.

Four hairpin probes with and without linkers, each having a different level of steric hindrance due to the reporters' proximity to the electrode surface to which the probes are bonded, were compared (Table 2). The length of linkers are adjusted by the TEG or/and overhang spacer in the 5-biotin labeled end. The overhang spacer is 9 thymidine (9T). The longitude size of biotinTEG is about 3 nm from MM2 calculation. See N. L. Allinger, J. Am. Chem. Soc., 99:8127-8134 (1977). Regarding the 9T linker, usually single stranded DNA is in a coiled state on the electrode when no force is applied. The coiled strand would stretch straight under a specific force, such as negative potential. Since the electrochemical detection is carried out under -200 mV, the 9T linker is stretched out at a much longer length instead of having a curved or lay-down structure. See A. M. van Oijen et al., Science, 301:1235-1238 (2003); U. Rant et al., Biophys. J., 90:3666-3671 (2006). Although the actual length of the 9T linker is not clear, it should be much longer than 3 nm under negative potential if taking the data from duplex DNA as 9 bp × 0.28 nm/bp. The size of HRP is about 4×6.7×11.8 nm from the crystal data. See G. I. Berglund et al., Nature, 417:463-468 (2002).

**Table 2 Oligonucleotide sequence for IL-8 hairpin probe with different linker length***

| Designation | Sequence (5' to 3') | 5'-label | 3'-label |
|---|---|---|---|
| IL-8 HPL0 | GAG GGT TGC TCA GCC CTC TTC AAA AAC TTC TCC ACA ACC CTC | Biotin | Fluorescein |
| IL-8HPL1 | GAG GGT TGC TCA GCC CTC TTC AAA AAC TTC TCC ACA ACC CTC | BiotinTEG | Fluorescein |
| IL-8 HPL2 | TTT TTT TTT GAG GGT TGC TCA GCC CTC TTC AAA AAC TTC TCC ACA ACC CTC | Biotin | Fluorescein |
| IL-8 HPL3 | TTT TTT TTT GAG GGT TGC TCA GCC CTC TTC AAA AAC TTC TCC ACA ACC CTC | BiotinTEG | Fluorescein |
| IL8CP | TTT TTT TAT GAA TTC TCA GCC CTC | Biotin | - |
| IL8DP | TTC AAA AAC TTC TCC ACA ACC CTC | - | Fluorescein |
| IL-8 HP | GAG GGT TGC TCA GCC CTC TTC AAA AAC TTC TCC ACA ACC CTC | Biotin | Fluorescein |
| S100A8 CP | TTT TTC CTG ATA TAC TGA GGA | Biotin | - |
| S100A8 DP | CAC TCG GTC TCT AGC AAT TTC | - | Fluorescein |
| S100A8 HP | GTG TCC TCT TTG AAC CAG ACG TCT GCA CCC TTT TTC CTG ATA TAC TGA GGA CAC | Biotin | Fluorescein |

| | | | |
|---|---|---|---|
| *Hairpin probe design is calculated by MFold free web software. See J. SantaLucia, *P Natl Acad Sci USA*, 95:1460-1465 (1998) and M. Zuker, *Nucleic Acids Research*, 31:3406-3415 (2003). | | | |

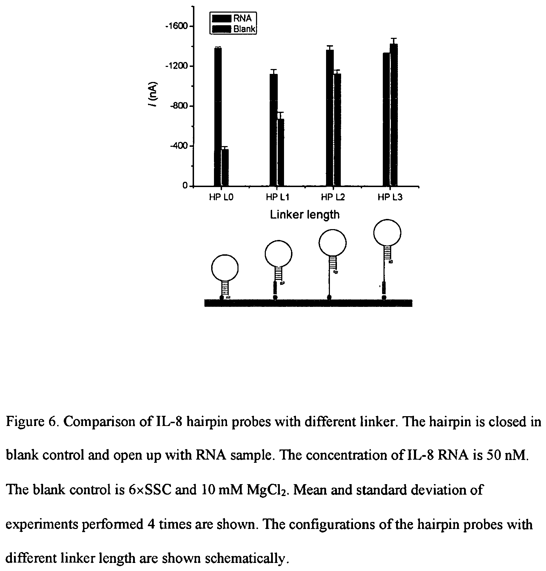

Figure 6 shows the effects from different levels of designed steric hindrance. For the TEG+9T probe with the longest linker (lowest steric hindrance), even when the hairpin is closed, the reporter is far away from the surface such that the complex between reporter and mediator can form and is effective. Therefore the measured output between no target and binding of target (IL-8) is small, as shown in the data set labeled "HP L3." When the steric hindrance is designed to be greater by removing the linker from the hairpin probe, the reporter is very close to surface when no target is bonded, preventing the formation of an effective mediator-reporter complex and therefore very low background noise is measured. After hybridization with target, the distance between reporter and surface increases and the complex is allowed to form and effectively regenerates the signal. The change of the signal is dramatic with a good signal to noise ratio.

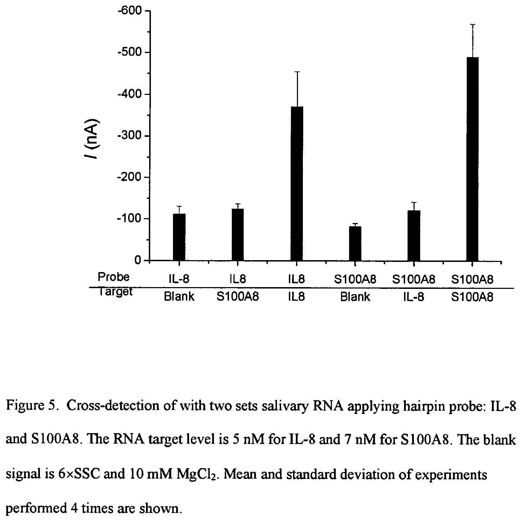

The specificity of the hairpin probes with 2 targets was tested, see Figure 5. For each probe, comparison between the complementary and non-complementary RNA targets has been carried out. Non-complementary targets give signal only 2 fold of standard deviation (SDV) higher than the blank signal. Signals of complementary targets are more than 20 SDV higher than the blank one. Both probes show good discrimination on the RNA level of 5 nM for IL-8 and 7 nM for S100A8.

### Control of SNR with hybridization efficiency

A major concern of the RNA sensor is the signal-to-noise ratio (SNR). In hairpin probes of the present invention, SNR depends on the open-to-closed ratio of hairpin probe. High SNR can be achieved by the well-closed status when no target bound and the full-open status after hybridization with targets. These closed-or-open states during recognition require high efficiencies for both the intra-molecular and inter-molecular hybridization.

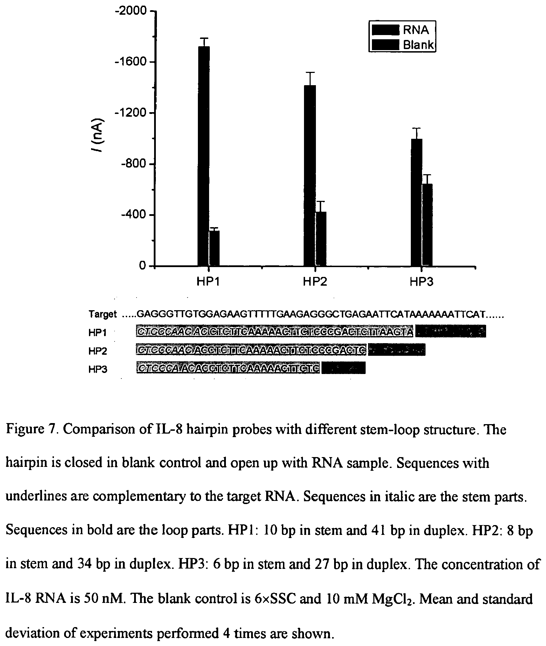

To increase the hybridization efficiency and optimize the SNR of the probes of the present invention, the hairpin structure was adjusted by changing the stem length and loop length. 3 hairpin probes with different stem-loop length have been studied (sequences listed in lower part of Figure 7). All 3 probes have the 3-end stem part complementary to the target RNA, together with the loop part. From the results shown in Figure 7, probe with the longest stem and duplex has the lowest signal for blank sample and highest signal for target sample. This result indicates that better closed state when no target and better open state when hybridized with target results in high signal-to-noise ratio. The probe with short stem and duplex doesn't have low background and high signal. Thus it is convenient to obtain high SNR with hairpin probe, since high hybridization efficiency simply benefits both the sensitivity and specificity. In contrast, it is difficult to find the optimized probe sequence with the traditional linear probes. Long sequence helps in the hybridization efficiency but generates high background, which results in conflicting effect for the sensitivity-specificity problem.

### Salivary RNA detection with hairpin probes and linear probes: spiked and non-spiked samples

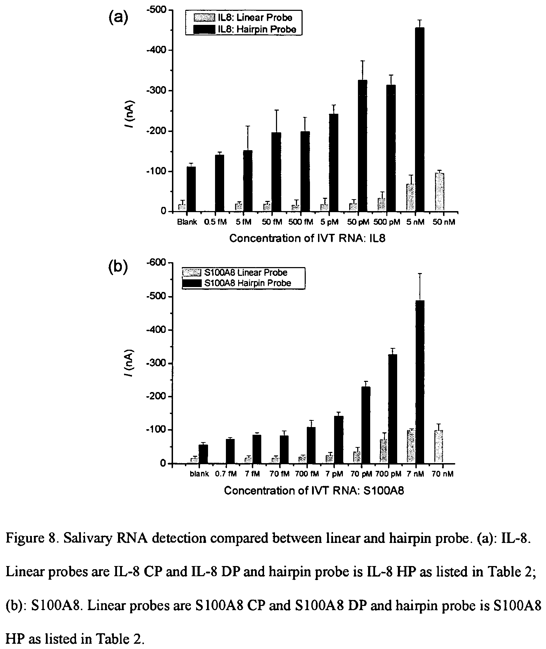

With the hairpin probes, one can consistently detect the salivary RNA biomarkers. Figure 8 shows the concentration relationship of the current signal. Both IL-8 and S100A8 exhibits good SNR with hairpin probes, but poor SNR with linear probes. It's interesting that the signal intensity is neither linear to concentration nor linear to the log of concentration. This phenomenon usually comes from complicated surface chemistry. In the present invention, there are several reactions integrated into a complex of total reaction, including the switching of hairpin probe, hybridization, protein recognition and enzymatic electrochemistry. It cannot be simply described in a linear relationship of target concentration. The limit of detection (LOD) of IL8 is about 1 fmol/L. For the linear probes, the LOD is only about 10 pmol/L. The LOD of S100A8 is about 1 fmol/L for hairpin probe and 10 pmol/L for the linear probes.

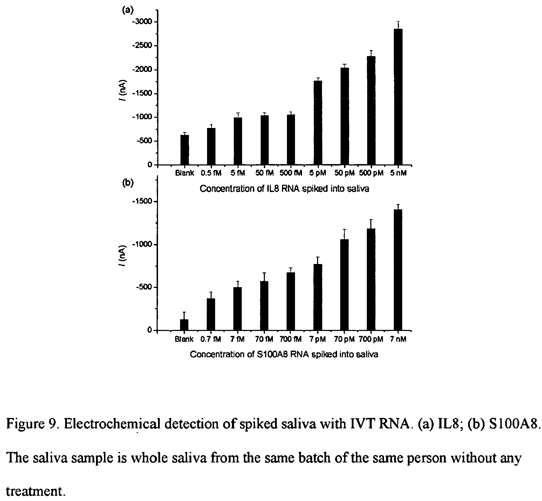

The salivary RNA biomarker was then detected with hairpin probe in spiked saliva. Data are shown in Figure 9. The RNA samples at different concentrations are spiked into whole saliva. Similar to the purified IVT RNA sample, spiked saliva also has low LOD. This indicates the hairpin probe can differentiate the specific target RNA even with huge number of interferents in saliva. The LOD is about 1 fM which could meet the requirement of real IL-8 salivary diagnostic.

Compared with the pure RNA sample results in Figure 8, an apparent signal increase was observed with saliva sample, especially the background level. The current of negative control for spiked sample is much higher than that of the IVT RNA. It was also observed that the signal level changes with different saliva sample even from the same person, compared with the stable signal level of pure RNA. The current for negative control varies from several hundreds of nA to several thousands of nA. A possible reason for the signal increase is the interfering components other than target RNA inside saliva, such as the mucin with high viscosity, which cause the non-specific binding of the following HRP or enhanced specific binding. See N. J. Park et al., Clin. Chem., 52:988-994 (2006). Since the concentrations of these interferents components in saliva vary with samples, the current intensity changes accordingly. Thus, in some embodiments, RNA detection is combined with lysis to remove other interferents.

### 1. Concepts of high signal-to-noise ratio with hairpin probe

In the present invention, the high signal-to-noise ratio comes from the combination of a sandwich-like detection and the hairpin probe. The concept of sandwich-like detection is the application of a mediator to form a complex, with a purpose of regenerating the HRP which amplify the signal. First, the target binds to the probe. Then a complex forms between reporter (fluorescein) labeled probe and mediator (anti-fluorescein-HRP) before detection. The excess mediator is removed by washing and TMB/H₂O₂ substrate is added to regenerate the HRP which amplifying the signal. See V. Gau et al., Methods, 37:73-83 (2005); J. C. Liao et al., J. Clin. Microbiol., 44:561-570 (2006). The signal level depends on both the amount and the activity of the complex. Because of the strong interaction between the metal electrode and the complex, the surface could serve as a restrictor for the formation of the complex, as well as an inhibitor for the activity of the reporter. This complex which is capable of amplify signal is referred to as the "effective complex." Only the effective complex generates amplified signal.

In conventional sandwich detection of nucleic acid, 2 linear oligonucleotide probes are employed: one is capture probe to immobilized the target on surface and the other is detector probe with a reporter to generate signal. See V. Gau et al., Methods, 37:73-83 (2005); E. Palecek et al., Anal. Chim. Acta, 469:73-83 (2002); H. Xie et al., Anal. Chem., 76:1611-1617 (2004). Therefore both the non-specific and specific target, independent of any mediator binding, would increase background and cause false-positive results. In order to increase the specificity, the steric hindrance effect was introduced into the hairpin structure. The hairpin probe is characteristic of its open-or-not two-state structure. When no target is bound, the hairpin probe stays in closed state thus the reporters cannot form effective complex with mediator because of the designed steric hindrance. After bound with a specific target, the probe turns into open state and a reporter forms an effective complex with the mediator, resulting a signal amplification. The steric hindrance design is simple and effective, removing a chemical reaction step from the original two probes design that decreases efforts in carrying out the experiments. Since the signal read-out in this work is only related to the complex formed between reporter and mediator, the target for detection is label-free. Label-free detection not only decreases the types of reagent use, but also makes real time and high throughput detection possible. It can be applied to micro-array and automatic *in situ* detection.

### 2. Principles for hairpin probe design

The basic idea of hairpin probe detection is the steric hindrance design which can specifically amplify the signal. There are 3 principles of the design: 1. Stable hairpin structure, which can be satisfied by stable stem part, *i.e*., long stem for hairpin (N. L. Goddard et al., Phys. Rev. Lett., 85:2400-2403 (2000)); 2. High hybridization efficiency, which can be satisfied by stable duplex part, i.e., long sequence for hybridization (N. L. Goddard et al., Phys. Rev. Lett., 85:2400-2403 (2000)); 3. High steric hindrance effect of the reporter which can be obtained by changing the linker length or introduce bigger mediator to increase the surface effect. By varying the hairpin probe structure, high SNR can be achieved by optimized steric hindrance effect.

Besides the hairpin probe design, there are two other methods increasing the surface steric hindrance effect. One is the surface density of probes. Densely packed hairpin probes have a higher crowding effect than the sparsely packed probes. It increases the restriction to both the binding of HRP and the activity of HRP. However, hybridization of target would be inhibited, too, under high surface concentration of probe. Sparse distribution of oligonucleotide gives high hybridization efficiency. There should be an optimized surface coverage for each probe and surface to get the best hybridization. As disclosed herein, the immobilization concentration of hairpin probe with 1×10⁻⁶ to 1×10⁻⁷ M gives good results.

The other factor is the electric potential applying to the electrode. Since the oliognucleotides are heavily negative charged, positive potential improves the hybridization and increases the attraction of the electrode to the strand. Negative potential forces the duplex to open and repel the strand from the surface. Oligonucleotide will lay down on electrode under high positive potential, while stretch out into the solution under negative potential. See U. Rant et al., Biophys. J., 90:3666-3671 (2006). When the probes lay down on electrode under positive potential, the surface steric hindrance to the probes in closed and open state would be almost the same, thus there is no differentiation for the complementary target binding. See R. G. Sosnowski, P Natl Acad Sci USA, 94:1119-1123 (1997). Therefore, negative potential is adopted from this point of view. However, a little bit positive potential will keep the un-bounded hairpin probes in closed state and stabilize the duplex with target. This helps to achieve high signal-to-noise ratio (data not shown). Meanwhile, negative potential will destroy the base pair interaction. If too high negative potential is applied, the hairpin probe would open even when no target is bound. See F. Wei et al., Langmuir, 22:6280-6285 (2006). The best potential for amperometric detection is very important and highly sensitive to the sequence composition of hairpin probe. See F. Wei et al., Langmuir, 22:6280-6285 (2006). As disclosed herein, the detection under negative potential (-200 mV) gives good SNR. One skilled in the art may readily optimize the potential for a given application.

### 3. Consideration for signal-to-noise ratio

Furthermore, compared with the traditional conformational-based detection which are usually signal-off processes, the detection of the present invention is a signal-on process. See C. H. Fan et al., P Natl Acad Sci USA, 100:9134-9137 (2003). Signal-on process detects an increase of the signal in a low background value, while signal-off process detects a decrease of the signal a high background value. Usually a measurement at high value has a larger error than that of lower value, so a signal-on process has a more steady background noise level. Furthermore, the dynamic range for the decrease of the signal is limited by the original background value in the signal-off process. Therefore, the signal-on process has a higher limit of detection, less measurement error, as well as more convenient for commercial use because it has less signal processing steps than signal-off process.

### Example 2: Electrochemical detection of salivary mRNA employing a hairpin probe (HP)

The probe was designed based on the principle that steric hindrance (SH) suppresses unspecific signal and generates a signal-on amplification process for target detection. The stem-loop configuration brings the reporter end of the probe into close proximity with the surface and makes it unavailable for binding with the mediator. Target binding opens the hairpin structure of the probe, and the mediator can then bind to the accessible reporter. Horseradish peroxidase (HRP) was utilized to generate electrochemical signal. This signal-on process is characterized by a low basal signal, a strong positive readout, and a large dynamic range. The SH is controlled via hairpin design and electrical field. By applying electric field control to hairpin probes, the limit of detection of RNA is about 0.4 fM, which is 10,000-fold more sensitive than conventional linear probes. Endogenous IL-8 mRNA is detected with the HP, and good correlation with the qPCR technique is obtained. The resultant process allows a simple setup and by reducing the number of steps it is suited for the point-of-care detection of specific nucleic acid sequences from complex body fluids such as saliva.

### Introduction

Molecular analysis of body fluids provides the potential for early cancer detection and subsequent increased treatment efficacy (Mandel, I.D. (1990) Journal of Oral Pathology & Medicine, 19, 119-125; Mandel, I.D. (1993) Journal of the American Dental Association, 124, 85-87; Wong, D.T. (2006) Journal of the American Dental Association, 137, 313-321). Molecular markers released from tumors find their way into blood and/or other body fluids, and specific detection of biomarkers may enable disease identification in a non-invasive and specific manner (Gormally et al. (2006) Cancer Research, 66, 6871-6876; Herr et al. (2007) Proceedings of the National Academy of Sciences of the United States of America, 104, 5268-5273). Saliva is easily accessible in a non-invasive manner, and can be collected with less patient discomfort relative to blood. In addition, the levels of interfering material (cells, DNA, RNA, and proteins) and inhibitory substances are lower and less complex in saliva than in blood. This advantage has recently been shown in a thorough study of oral cancer mRNA markers (Li et al. (2004) Clinical Cancer Research, 10, 8442-8450). mRNAs were identified through microarray and validated according to established guidelines (Pepe et al. (2001) Journal of the National Cancer Institute, 93, 1054-1061) by quantitative PCR (qPCR). Detecting salivary mRNA biomarkers adds a new dimension to saliva as a valuable diagnostic fluid. In this study, we aimed to develop a unique methodology for on-site testing of salivary mRNA.

Electrochemistry is an excellent candidate for a point-of-care diagnostic method for RNA detection (Hahn et al. (2005) Bioelectrochemistry, 67, 151-154), not only because of its high sensitivity but also because of the simplicity of the instrument (Liao and Cui (2007) Biosensors & Bioelectronics, 23, 218-224; Wei et al. (2005) Journal of the American Chemical Society, 127, 5306-5307; Wei et al. (2006) Langmuir, 22, 6280-6285; Wei et al. (2003) Biosensors & Bioelectronics, 18, 1157-1163; Wei et al. (2003) Biosensors & Bioelectronics, 18, 1149-1155). However, due to the low concentration (~fM) of salivary biomarkers and the complex background of saliva, conventional electrochemical amperometric detection methods do not meet the clinical diagnostic requirement of high signal-to-background ratio (SBR) for direct RNA detection in saliva.

Recently, Plaxco's group reported a novel method of applying redox-labeled hairpin probes to enable oligonucleotide detection in various body fluids including serum and urine (Lubin et al. (2006) Analytical Chemistry, 78, 5671-5677; Xiao et al. (2006) Proceedings of the National Academy of Sciences of the United States of America, 103, 16677-16680). This method successfully demonstrated the use of hairpin probes (HP) as a switch between closed and open status during an electrochemical reaction. The results provided significant improvements in both sensitivity and specificity. In the context of saliva diagnostics, low copy-numbers of RNA biomarkers in saliva demand highly sensitive sensors to detect signal above background noise. Herein, we propose a method that couples an enzymatic amplification process with a target-induced conformational change based on an HP probe. This HP comprises a loop component with a sequence complementary to the target and a stem component labeled with a reporter at one end. Without target binding, the proximity to the sensor surface creates steric hindrance (SH), which inhibits signal amplification by preventing mediator access to the probe reporter label. This built-in SH is removed after the bio-recognition component verifies the target specificity, making the reporter label accessible to the mediator-peroxidase conjugate and generating a current signal. Therefore, only the specific target can generate an amplified current, even if present in low copy numbers and in a complex mixture. The SH effect is controllable in this HP-based electrochemical sensor by optimizing probe design and the surface electrical field. Our selective amplification method suppresses non-specific signal to background levels, overcoming key hurdles in developing point-of-care nucleic acid detection systems for salivary RNA markers and for other general use.

### Materials and Methods

### Oligonucleotide probes and RNA

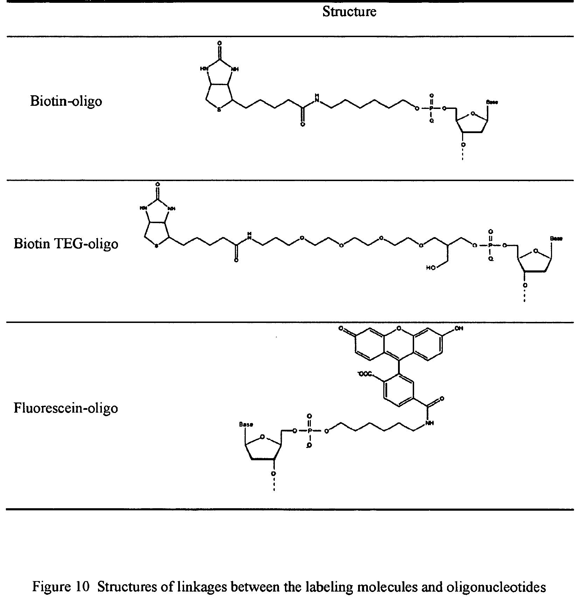

HPLC-purified oligonucleotides were custom synthesized (Operon Inc., Alabama, USA). The probe sequence allowed for the formation of a hairpin structure. The loop and half of the hairpin stem (3' end) contained target recognition sequences, and HPs were labeled with biotin or biotin-TEG on the 5' end and with fluorescein on the 3' end (detailed structures are shown in Figure 10). The biotin label bound to streptavidin as an anchor to the chip surface, and the fluorescein label allowed for binding of the signal mediator. The present inventors investigated the following configurations of the 5' linker from the probe to the chip surface: biotin link, biotin-TEG, biotin-9 thymidines (T₉), and biotin-TEG-T₉. Biotin-TEG had an extra spacer with mixed polarity based on triethylene glycol containing oxygen atoms connecting the biotin and the oligo chain. Different spacing designs may confer better accessibility of the biotin to the streptavidin, and could serve as an adjustable length linker for the SH effect.

Interleukin 8 (IL-8) mRNA (NM_000584)(St John et al. (2004) Archives of Otolaryngology-Head & Neck Surgery, 130, 929-935) has been proposed as a salivary biomarker for oral cancer and was selected for detection. For the purpose of establishing the validity of the method, in vitro transcribed (IVT) IL-8 RNAs were used as a target for standard quantitative measurements. Details of IVT RNA generation are described in the supplementary materials II section. Endogenous mRNAs were detected from clinical samples. For detecting endogenous IL-8 from saliva samples, a lysis process was carried out by mixing the saliva 1:1 with AVL viral lysis buffer (QIAGEN, California, USA) for 15 min at room temperature. Details of saliva collection and qPCR measurements are described in the supplementary materials III-IX.

### Generation of in vitro-transcribed RNA for the RNA markers

Two mRNA targets were selected for the detection. Interleukin 8 (IL-8) (mRNA, NM_000584) (St John et al. (2004) Archives of Otolaryngology-Head & Neck Surgery, 130, 929-935) has been proposed as a candidate biomarker for oral cancer. S100 calcium-binding protein A8 (S100A8) (mRNA, NM_002964), which highly expressed in saliva, was used as a reference on each electrochemical sensor and shows no oral cancer relevance.

For the purpose of method establishment, *In vitro* transcribed (IVT) RNAs of IL-8 and S100A8 were used as target for detection in this study. The IVT RNAs were generated in two steps: first was to generate templates for in vitro transcription using conventional RT-PCR, in which the primers having 20 base core T7 promoter sequence at the 5' end of the forward primers. For IL-8, the forward primer is 5'-CTAATACGACTCACTATAGGGaaggaaaactgggtgcagag-3', and the reversed primer is 5'-attgcatctggcaaccctac-3'. For S100A8, the forward primer is 5' CTAATACGACTCACTATAGGGatcatgttgaccgagctgga-3', and the reversed primer is 5'-gtctgcaccctttttcctga-3'. The products were 177 bp and 159 bp double strands DNA, respectively. The conventional RT-PCR was conducted with total oral squamous cell carcinoma (OSCC) cell line RNA as template and cDNA was synthesized in 20 µl of reverse transcription reaction mix with 50 U MuLV reverse transcriptase (Applied Biosystems), 20 U RNAse Inhibitor (Applied Biosystems), 10 mM dNTPs and 5 nmol random hexamers. The mix was first incubated at 25°C for 10 min, then reverse transcribed at 42°C for 45 min followed by a final inactivation of RT at 95°C for 5 min and cooling at 4°C for 5 min. One micro litter cDNA was used in a 20 µl PCR reaction with 400 nM primers. The PCR reaction was carried out by the following protocol: 95°C for 3 min followed by 40 cycle of 95°C for 30s, 60°C for 30s, 72°C for 30 s, and final extension at 72°C for 7 min. RT-PCR products were checked on a 2% agarose gel stained with ethidium bromide. The second step was to generate the IVT RNAs. In vitro transcription was performed using T7 MEGAshort transcribe kit (Invitrogen) according to manufacturer's instruction. Briefly, 8 µl PCR products from the first step was in vitro transcribed at 37°C for 3 hrs and followed by 2 µl rDNase1 (Invitrogen) treatment for additional 20 min. The resultant single strand RNA transcripts were purified with cleaned up (Arcturus, Mountain View, CA). The recombinant RNAs were quantified with Nanodrop spectrometry for quantity and A260/A280 ratio. Resultant RNA were dissolved in RNase-free distilled water (Invitrogen) with baker's yeast tRNA (30 µg/ml, Roche) as carrier.

### Saliva collection

Un-stimulated whole saliva was collected according to our published protocol (Li, Yang 2004). Briefly, all saliva samples were collected while kept on ice. Upon collection, RNAlater (QIAGEN, Valencia, CA) at room temperature was added into the saliva samples at a 1:1 (volume) ratio and mixed by vortexing. RNAlater at 1:1 ratio mixed with whole saliva and samples stored at -80°C provides prompt and adequate inhibition of salivary RNA degradation. The sample aliquots were stored at -80°C for later use.

### Total salivary RNA extraction

Total RNA was extracted according to the following procedures: frozen saliva preserved in RNAlater was thawed on ice and total RNA was extracted using viral mini kit (QIAGEN) according to manufacturer's instructions with the following exception: In order to make it comparable to previously reported procedure (Li, Yang, 2004), two times starting volume of saliva RNAlater mix (2x560 ul) was used to compensate for the saliva dilution by RNAlater. The resultant total RNA was eluted in 40 µl of elution buffer, and was treated with rDNase 1 (Ambion, Austin TX) in a solution containing 40 µl RNA, 4.5 µl 10× DNase I buffer, 0.5 µl rDNaseI for 30 minutes at 37°C to remove any genomic DNA contamination. After clean up with DNase inactivator, up to 35 µl total RNA were recovered and frozen at - 80°C until use.

### Primer design

Intron-spanning primer pairs with melting temperatures around 60 °C for IL8 were designed with the primer3 program. OF and OR are primers for RT-PCR and IF and IR were designed for qPCR.

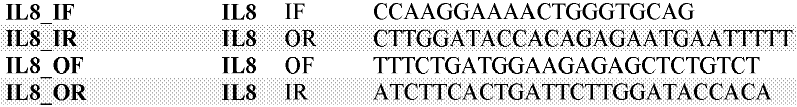

### RT-PCR pre-amplification

One-step RT and PCR pre-amplification were performed in 20-40 µl reactions with the SuperScript III Platinum One-Step qRT-PCR System (Invitrogen, Carlsbad, CA), primer concentrations were 300 nM for all targets. The reactions were set up utilizing the BioMek 3000 liquid handling platform into 96-well plates on PCR plate cooler and then performed with the following program: 1 min at 60 °C, 15 min at 50 °C, 2 min at 95 °C, and 15 cycles of 15 s at 95 °C, 30 s at 50 °C and, 10 s at 60 °C and 10 s at 72 °C, with a final extension for 5 min at 72 °C and cooling to 4 °C.

### Cleanup of pre-amplification reaction

Immediately after RT-PCR, 5 µl of the reaction were treated with 2 µl of Exo-SAP-IT^{®} (USB, Cleveland, OH) for 15 min at 37 °C to remove excess primers and dNTPs, and then heated to 80 °C for 15 min to inactivate the enzyme mix. The reaction was diluted with nuclease free water by a factor of 40 unless reported otherwise. Dilution factors refer to the volume of pre-amplificate prior to Exo-SAP-IT treatment.

### Quantitative real-time PCR

All reaction were set up by automation using the BioMek 3000 liquid handling platform into 96-well plates.A 4 µl aliquot of the preamplificate dilution was amplified with 300 nM of a pair of semi-nested assays. Reactions of 10 µl with the SYBR Green Power reaction mix (Applied Biosystems (AB), Foster City, CA) were set up on ice and carried out in a SDS 7500 Fast instrument (AB). After 10 min activation of the polymerase at 95 °C, 40 cycles of 15 s at 95 °C and 60 s at 60 °C were performed, followed by melting curve analysis.

### qPCR Analysis

The automatic baseline setting of the 7500 Fast System v1.3.1 software (AB) was used for qPCR analysis.

### Surface Preparation

The surface preparation of the gold electrochemical sensor was performed as follows(Gau et al. (2001) Biosensors & Bioelectronics, 16, 745-755; Gau et al. (2005) Methods, 37, 73-83):

*Probe immobilization:* The gold electrodes were pre-coated with a self-assembled monolayer of mercaptoundecanoic acid (MUDA), terminated by a carboxyl group (18). The gold surface was activated by a 4 µL mixture of 50% 1-ethyl-3-(3-dimethylaminopropyl) carbodiimide hydrochloride (EDC, Biacore Inc., New Jersey, USA) and 50% N-hydroxysuccinimide (NHS) (Biacore) for 10 min. The sensors were rinsed with DI water (18.3 MΩ•cm) and dried with nitrogen gas. A total of 4 µL of 5 mg/mL amine-PEO2-Biotin labeling reagent (Ez-Biotin) (Pierce Inc., Illinois, USA) was loaded to the gold surface, followed by rinsing and drying. Ethanolamine-HCl (1.0 M, pH 8.5, Biacore) was loaded for inactivation of the un-reacted EDC/NHS activated surface. Next, 0.5 mg/mL streptavidin (VWR Corp., California, USA) in PBS (pH 7.2, Invitrogen, California, USA) was incubated on the electrode for 10 min to produce streptavidin-coated electrodes. A total of 4 µL of 5'-biotinylated and 3'-fluorescein dual-labeled HP in Tris-HCl buffer (pH 7.5, Invitrogen, California, USA) was immobilized onto the electrodes for 30 min via the interactions between streptavidin on the surfaces and the biotin label on the probes. The surface density of the oligo probe achieved using this immobilization strategy was reported to be ~ 3.4 × 1012 molecules/cm2 (Su et al. (2005) Langmuir, 21, 348-353). Excessive HP was removed by a thorough rinse with DI water and dried with nitrogen gas.

*Target Hybridization*: The surface was incubated for 5 min with the target-containing sample prepared in 6x saline-sodium citrate buffer (6x SSC, 0.09 M sodium citrate, with 0.9 M NaCl, pH 7.0, Invitrogen, California, USA) with the addition of 10 mM MgCl₂ (Sigma Corp., Missouri, USA). During hybridization, a cyclic square-wave electric field was applied at 30 cycles of +200 mV for 1s and -300 mV for 9s. After hybridization, the electrodes were rinsed with DI water and dried with nitrogen gas.

### Electrochemical detection

The electrochemical readout was performed using an electrochemical workstation according to the manufacturer's instructions. Briefly, anti-fluorescein-HRP (Roche, Indiana, USA) diluted in PBS with 0.5% casein blocking buffer (Blocker Casein in PBS, Pierce, pH 7.4) was added to the fluorescein label on the HP or the detector probes. Then, 3, 3', 5, 5' tetramethylbenzidine low activity (TMB/H₂O₂, Neogen Corp., Kentucky, USA) substrate was loaded, and amperometric detection was carried out by applying -200 mV potential vs. gold to each electrode unit, followed by parallel signal read-out after 60 s of equilibration (Gau et al. (2001) Biosensors & Bioelectronics, 16, 745-755; Gau et al. (2005) Methods, 37, 73-83).

The electrochemical sensor was a 16-unit gold array. For each unit, there were three electrodes including the working electrode (WE), counter electrode (CE) and reference electrode (RE) (Gau et al. (2005) Methods, 37, 73-83). The reference electrode was determined to be +218 mV vs. SCE by measuring cyclic voltammetric curves of 0.1 mM [Fe(CN)₆]^{3-/4-}. All electric potentials described in this report are in reference to the gold reference electrode (+218 mV vs. SCE). The advantages of this small electrode array are that the signal read-out of the 16 electrodes can be obtained simultaneously, and only 4 µL of sample solution is needed for detection. In our experiments, the electrochemical signal was the current generated by the redox of the HRP reporter enzyme. TMB continually regenerated reduced HRP via a 2-electron step, which amplified the current signal. The current was proportional to the surface concentration of hybridized target (Gau et al. (2005) Methods, 37, 73-83). All experiments were performed at room temperature.

### Results and Discussion

### 1. Hairpin-induced specific amplification

Detection of a specific target using the current approach was accomplished via a combination of sandwich-like signal amplification by HRP and TMB/H₂O₂ as well as selective hybridization by the HP design. This method was based on the SH effect: the surface near the HP inhibits the HRP conjugate binding to target-free probes. Therefore, the distance between the surface and reporter label on the probe was a key factor to the detection process. Upon target binding, the HP opened and the reporter was away from the surface, resulting in reduced restriction from the surface. Conjugated HRP bound to the fluorescein and generated current, constituting a signal-on process.

Four IL-8 specific HPs were compared with and without 5'-linkers, which exhibited different levels of SH due to varying distances between the reporters and the electrode surface (Table 3). The length and flexibility of linkers were adjusted by the length of the TEG or an overhang spacer (T₉) at the 5'-biotin labeled end. The longitudinal size of biotin-TEG was approximately 3 nm from molecular mechanics calculations (MM2) (Allinger, N.L. (1977) Journal of the American Chemical Society, 99, 8127-8134). Single stranded DNA was in a coiled state on the electrode when no force was applied. The coil was probably stretched to permit conjugate binding when the electrochemical detection was carried out at negative potential (Rant et al. (2006) Biophysical Journal, 90, 3666-3671; van Oijen et al. (2003) Science, 301, 1235-1238). Although the exact length of the T₉ linker is not known, it was likely >3 nm under the negative potential, if duplex DNA is 9 bp x 0.28 nm/bp. The size of HRP is approximately 4 x 6.7 x 11.8 nm, according to protein crystal data (Berglund et al. (2002) Nature, 417, 463-468).

Figure 6 shows the SH effects from different HP designs. For the probe with the longest linker (TEG-T₉), the fluorescein was far away from the surface even when the hairpin was closed. The mediator complex was formed, and SH effect was very small. Hybridization to the target only increased the distance of the HRP complex from the electrodes. Therefore, the signal decreased upon binding, and recognition resulted in a very weak signal-off process (Fig. 6). Signals with bound target were at similar levels for all four probes, and the blank signal decreased with decreasing linker length. For the HP without a linker, the reporter was very close to the surface in the closed state. Therefore, the SH effect was very strong, and the lowest background was observed (SBR = 8:1).

### 2. Specificity

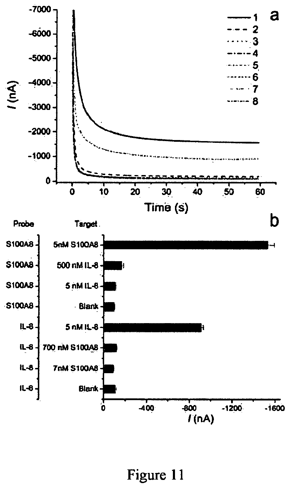

The specificity of HP without a linker was tested with cross-detection of 2 targets, and the results are shown in Figure 11. As a reference control, we used the mRNA for S100 calcium-binding protein A8 (S100A8 mRNA, NM_002964), which is highly expressed in saliva and has no oral cancer relevance. For each probe, a comparison between the complementary and non-complementary IVT RNA target was carried out at concentrations of 5 nM and 500 nM for IL-8, and 7 nM and 700 nM for S100A8. Even non-complementary targets that were over-expressed by 100-fold gave little signal increase for the IL-8- and S100A8-specific probes. Complementary target signals were >20 standard deviations (SDV) higher than the blank control. Both probes showed good RNA discrimination for 5 nM of IL-8 and 7 nM of S100A8.

### 3. Control ofSBR with hybridization efficiency

A major concern of the RNA sensor is the SBR. In the current HP designs, the SBR depended on the ratio of the numbers with an open or closed HP. Background levels were associated with the closed state when no specific target was bound, and signal was generated from the open state after target hybridization. These closed or open states during recognition required high efficiencies for both the intra-molecular and inter-molecular hybridization.

To increase hybridization efficiency and optimize the SBR of this sensor, we modified the hairpin structure by changing both the stem and loop length. Three HPs with different stem-loop lengths were studied (sequences listed in Table 4). In all three probes, the 3'-end stem component was complementary to the target RNA, together with the loop. The probe with the short stem (6 bp) and the duplex (21 + 6 bp) had a high background and low signal (HPS3 in Fig. 7). The probe with the longest stem (10 bp) and the duplex (10 + 31 bp) had the lowest blank signal and the highest signal for target (HPS 1), indicating a better closed state when no target was bound and a better open state when hybridized with target. Complementary HP sequences included both the whole loop and half of the stem, providing lower free energy after target hybridization. Thus, once target was bound to the loop, even the very long stem could be opened due to its complementary sequence to the target. Since high hybridization efficiency benefits both the sensitivity and specificity, a good SBR was achieved. In contrast, it is difficult to determine the optimized probe sequence with the traditional linear probe (LP) (Liao et al. (2006) Journal of Clinical Microbiology, 44, 561-570). The long sequence was beneficial to the hybridization efficiency, but generates high background.

### 4. Detection of spiked RNA in saliva:

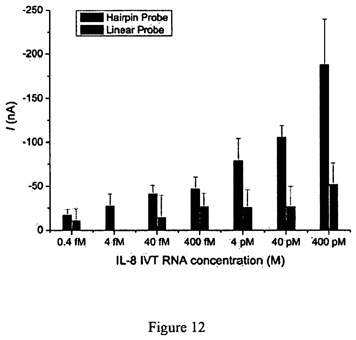

With proper HP design and cooperation from the SH effect, salivary RNA biomarker sequences can be detected over a wide dynamic range of target concentration. Fig. 12 shows the relationship between the concentration and the current signal in buffer. For comparison, the original system with two LPs for each target was also examined, using previously published methods (Liao et al. (2006) Journal of Clinical Microbiology, 44, 561-570). Briefly, both probes were designed to be complementary to adjacent stretches of the target sequence. The 'capture probe' was immobilized on the electrode with a 5' end biotin label. The 'detector probe' had a 3' fluorescein label to bind with the anti-fluorescein-HRP. Our results show that good SBR for detecting IL-8 was obtained with HP, but poorer performance was seen with LP.

The limit of detection (LOD) was defined as the concentration with a signal of at least 2 SDV above the background level. According to the criteria, the LOD for HP was about 0.4 fM. For the LPs, the LOD of IL-8 was about 400 pM, which is about 10,000-fold higher than for the HP (Fig. 12).

### 5. Detection of endogenous mRNA in saliva:

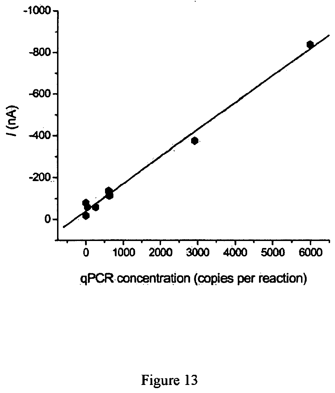

We then proceeded to detect endogenous IL-8 mRNA in saliva samples. Changes in signal levels between different saliva samples were observed. IL-8 mRNA in seven clinical saliva samples were measured using the present optimized HP design. Since endogenous mRNA in saliva is combined with other macromolecules which mask detection, a lysis procedure was carried out before the electrochemical assay to release masked RNA. A good correlation was observed between the electrochemical signals for saliva samples and the qPCR results, as shown in Figure 13. Higher electrochemical signals were observed in the saliva samples containing a higher level of IL-8 mRNAs as determined by qPCR measurement. In addition to the PCR measurement, these results support the existence of mRNA in saliva. The results also show that endogenous mRNA can be detected in saliva by an electrochemical method without PCR amplification, which meets the sensitivity requirement for point-of-care salivary diagnostics.

For detection of DNA oligonucleotides using various electrochemistry-associated methods, LOD in the fM range have been achieved. These methods include nano-particle-linked secondary probes (Park et al. (2002) Science, 295, 1503-1506), anodic stripping voltammetry of silver nanoparticles deposited in a multi-step reduction process (Hwang and Kwak (2005) Anal Chem, 77, 579-584), and electronic DNA sensors based on target-induced strand displacement mechanisms (Xiao et al. (2006) Proceedings of the National Academy of Sciences of the United States of America, 103, 16677-16680). mRNA has a longer sequence and more complicated secondary structure than oligos. To capture specific mRNA targets, a characteristic fragment of mRNA must be chosen carefully. Secondary mRNA structure may reduce hybridization between the capture probe and the target. In this study, the inventors chose the mRNA fragment with minimal secondary structure, as calculated by the Mfold web server (Zuker, M. (2003) Nucleic Acids Research, 31, 3406-3415). Probe design also required thorough consideration of loop sequence, stem length, and probe secondary structure. Considering the intrinsic 2-D or 3-D structure of the RNA, the following principles were applied for both linear and hairpin probe design:
(1) Affinity of probe to the target mRNA: mRNA secondary structure and secondary structure of probe sequences which are complimentary to the target RNA, including quadruplex and hairpin, were considered. Sequences without stable secondary structures were selected based on quadruplex and M-fold calculations. Formation of self-dimers and hybridization stability also were considered based on thermodynamic calculations.
(2) For optimal hairpin probe performance, half of the stem (3' end), together with the loop was designed to be complementary to the target RNA. Since the 5' end of the stem was immobilized onto the surface via biotin-streptavidin for all the HPs in this study, only the 3' stem was free during the hybridization process. Sharing the 3' end of the stem with the loop for duplex formation resulted in higher hybridization efficiency and more changes in the SH effect.

In summary, the present inventors developed an effective method for electrochemical detection of mRNA using HP with high sensitivity, high specificity, and a large dynamic range (fM - nM in buffer system and spiked saliva). The inventors also demonstrated that this technique works well for directly detecting endogenous mRNA without the need for PCR amplification.

**Table 3. Oligonucleotide sequences for IL-8 and S100A8.**

| Designation | Sequence (5' to 3') | 5'-label | 3'-label |
|---|---|---|---|
| IL-8 HPL0^{*†} | GAG GGT TGC TCA GCC CTC TTC AAA AAC TTC TCC ACA ACC CTC | Biotin | Fluorescein |
| IL-8 HPL1^{*†} | GAG GGT TGC TCA GCC CTC TTC AAA AAC TTC TCC ACA ACC CTC | BiotinTEG | Fluorescein |
| IL-8 HPL2^{*†} | TTT TTT TTT GAG GGT TGC TCA GCC CTC TTC AAA AAC TTC TCC ACA ACC CTC | Biotin | Fluorescein |
| IL-8 HPL3^{*†} | TTT TTT TTT GAG GGT TGC TCA GCC CTC TTC AAA AAC TTC TCC ACA ACC CTC | BiotinTEG | Fluorescein |
| IL-8 CP‡ | *TTT TTT TAT GAA TTC TCA GCC CTC* | Biotin | - |
| IL-8 DP‡ | *TTC AAA AAC TTC TCC ACA ACC CTC* | - | Fluorescein |
| IL-8 HP^{*†} | GAG GGT TGC TCA GCC CTC TTC AAA AAC TTC TCC ACA ACC CTC | Biotin | Fluorescein |
| S100A8 HP^{*†} | GTG TCC TCT TTG AAC CAG ACG TCT GCA CCC TTT TTC CTG ATA TAC TGA GGA CAC | Biotin | Fluorescein |

| | | | |
|---|---|---|---|
| * Hairpin probe design was calculated by MFold free web server (27, 28). † The target recognition sequences are listed in italic font. The stem sections of the hairpins are underlined. ‡ IL-8 CP is the capture probe with biotin label in dual probes detection. IL-8 DP is the detect probe with fluorescein label in dual probes detection. | | | |

**Table 4. Oligonucleotide sequences for IL-8 HP with different stem-loop structures.**

| Designation | Sequence (5'to 3') | Stem (bp) | Loop (nt) | Duplex (bp) |
|---|---|---|---|---|
| II-8 HPS1^{*†‡} | GAG GGT TGT GAT GAA TTC TCA GCC CTC TTC AAA AAC TTC TCC ACA ACC CTC | 10 | 31 | 41 |
| IL-8 HPS2^{*†‡} | GAG GGT TGC TCA GCC CTC TTC AAA AAC TTC TCC ACA ACC CTC | 8 | 26 | 34 |
| IL-8 HPS3^{*†‡} | GAG GGT CTC TTC AAA AAC TTC TCC ACA ACC CTC | 6 | 21 | 27 |

| | | | | |
|---|---|---|---|---|
| * All the probes were double labeled with 5'-biotin and 3'-fluorescein. † Hairpin probe design was calculated by MFold free web server (27,28). ‡ The target recognition sequences are listed in italic font. The stem sections of the hairpins are underlined. | | | | |

## Claims

1. A hairpin probe for detecting an mRNA or DNA biomarker comprising a polynucleotide sequence that specifically hybridizes to the sequence of the mRNA or DNA biomarker, and a ligand for a receptor wherein the receptor comprises a detectable label, said hairpin probe having a first three-dimensional structure in the absence of the mRNA or DNA biomarker being bound thereto and a second three-dimensional structure in the presence of the mRNA or DNA biomarker being bound thereto, wherein the first three-dimensional structure is a stem-loop structure where the stem is formed by the hybridization of two regions of the hairpin probe, wherein the first three-dimensional structure inhibits or prevents the receptor from binding the ligand and the second three-dimensional structure allows the receptor to specifically bind the ligand, and wherein one of the two regions of the stem and the loop of the hairpin probe is complementary to a part of the sequence of the mRNA or DNA biomarker, wherein the probe is immobilized on an electrochemical substrate, and wherein the first three-dimensional structure places the ligand at a position near the substrate such that the receptor is inhibited or prevented from binding the ligand.

2. The probe of claim 1, wherein the detection signal from the detectable label is capable of being amplified.

3. The probe of claim 1 or 2, wherein the receptor is an antibody that specifically binds the ligand.

4. The probe of any one of the preceding claims, wherein the detectable label is fluorescein, streptavidin, or biotin.

5. The probe of any one of the preceding claims, wherein the detection signal is amplified by peroxidase, laccase, glucose oxidase, alkaline phosphatase, or urease.

6. The probe of any one of the preceding claims, wherein the biomarker is an IL-8 mRNA or DNA sequence.

7. The probe of any one of the preceding claims, wherein the polynucleotide sequence is selected from Tables 2-4.

8. A method for detecting an mRNA or DNA biomarker in a sample, the method comprising:
contacting the probe of any one of the preceding claims with the sample and the receptor, and detecting the presence or absence of a complex between the ligand and the receptor,
wherein the presence of the complex indicates the presence of the mRNA or DNA biomarker in the sample and the absence of the complex indicates the absence of the mRNA or DNA biomarker in the sample.

9. The method of claim 8, wherein the sample is a saliva sample.

10. The method of claim 8 or 9, wherein the biomarker is an IL-8 mRNA or DNA sequence.

## Patentansprüche

1. Haarnadel-Sonde zum Nachweisen eines mRNA- oder DNA-Biomarkers, umfassend eine Polynukleotidsequenz, die spezifisch mit der Sequenz des mRNA- oder DNA-Biomarkers hybridisiert, und einen Liganden für einen Rezeptor, wobei der Rezeptor eine detektierbare Markierung umfasst, wobei die Haarnadel-Sonde eine erste dreidimensionale Struktur in Abwesenheit des mRNA- oder DNA-Biomarkers, der daran gebunden ist, und eine zweite dreidimensionale Struktur in Anwesenheit des mRNA- oder DNA-Biomarkers, der daran gebunden ist, aufweist, wobei die erste dreidimensionale Struktur eine Stamm-Schleifen-Struktur ist, bei welcher der Stamm durch die Hybridisierung von zwei Regionen der Haarnadel-Sonde gebildet wird, wobei die erste dreidimensionale Struktur die Bindung des Liganden an den Rezeptor inhibiert oder verhindert und die zweite dreidimensionale Struktur es dem Rezeptor ermöglicht, den Liganden spezifisch zu binden, und wobei eine von den beiden Regionen von dem Stamm und der Schleife der Haarnadel-Sonde komplementär zu einem Teil der Sequenz des mRNA- oder DNA-Biomarkers ist, wobei die Sonde auf einem elektrochemischen Substrat immobilisiert ist und wobei die erste dreidimensionale Struktur den Liganden in eine Position in der Nähe des Substrates derart platziert, dass der Rezeptor inhibiert wird oder an der Bindung des Liganden gehindert wird.

2. Sonde nach Anspruch 1, wobei das Detektionssignal von der detektierbaren Markierung amplifiziert werden kann.

3. Sonde nach Anspruch 1 oder 2, wobei der Rezeptor ein Antikörper ist, der spezifisch den Liganden bindet.

4. Sonde nach einem der vorhergehenden Ansprüche, wobei die detektierbare Markierung Fluorescein, Streptavidin oder Biotin ist.

5. Sonde nach einem der vorhergehenden Ansprüche, wobei das Detektionssignal durch Peroxidase, Laccase, Glucoseoxidase, alkalische Phosphatase oder Urease verstärkt wird.

6. Sonde nach einem der vorhergehenden Ansprüche, wobei der Biomarker eine RNA- oder DNA-Sequenz von IL-8 ist.

7. Sonde nach einem der vorhergehenden Ansprüche, wobei die Polynukleotidsequenz aus den Tabellen 2 - 4 ausgewählt ist.

8. Verfahren zum Detektieren eines mRNA- oder DNA-Biomarkers in einer Probe, wobei das Verfahren umfasst:
Inkontaktbringen der Sonde nach einem der vorhergehenden Ansprüche mit der Probe und dem Rezeptor und Detektieren der Anwesenheit oder der Abwesenheit eines Komplexes zwischen dem Liganden und dem Rezeptor,
wobei die Anwesenheit des Komplexes die Anwesenheit des mRNA- oder DNA-Biomarkers in der Probe anzeigt und die Abwesenheit des Komplexes die Abwesenheit des mRNA- oder DNA-Biomarkers in der Probe anzeigt.

9. Verfahren nach Anspruch 8, wobei die Probe eine Speichelprobe ist.

10. Verfahren nach Anspruch 8 oder 9, wobei der Biomarker eine mRNA- oder DNA-Sequenz von IL-8 ist.

## Revendications

1. Une sonde en épingle pour détecter un biomarqueur ARNm ou ADN comprenant une séquence polynucléotide qui s'hybride spécifiquement avec la séquence du biomarqueur ARNm ou ADN, et un ligand pour un récepteur dans laquelle le récepteur comprend un marqueur détectable, ladite sonde en épingle ayant une première structure tridimensionnelle en l'absence du biomarqueur ARNm ou ADN qui lui est lié et une deuxième structure tridimensionnelle en présence d'un biomarqueur ARNm ou ADN qui lui est lié, dans laquelle la première structure tridimensionnelle est une structure tige-boucle où la tige est formée par l'hybridation de deux zones de la sonde en épingle, dans laquelle la première structure tridimensionnelle entrave ou empêche le récepteur de créer une liaison avec le ligand, et la deuxième structure tridimensionnelle permet au récepteur de créer une liaison spécifique avec le ligand et dans laquelle une des deux zones de la tige et de la boucle de la sonde en épingle est complémentaire à une partie de la séquence du biomarqueur ARNm ou ADN, dans laquelle la sonde est immobilisée sur un substrat électrochimique, et dans laquelle la première structure tridimensionnelle place le ligand dans une position proche du substrat de telle façon que le récepteur est entravé ou empêché de créer une liaison avec le ligand.

2. La sonde selon la revendication 1, dans laquelle le signal de détection du marqueur détectable est susceptible d'être amplifié.

3. La sonde selon la revendication 1 ou 2, dans laquelle le récepteur est un anticorps qui lie spécifiquement le ligand.

4. La sonde selon l'une quelconque des revendications précédentes, dans laquelle le marqueur détectable est de la fluorescéine, de la streptavidine, ou de la biotine.

5. La sonde selon l'une quelconque des revendications précédentes, dans laquelle le signal de détection est amplifié par de la peroxydase, de la laccase, de la glucose oxidase, de la phosphatase alcaline, ou de l'uréase.

6. La sonde selon l'une quelconque des revendications précédentes, dans laquelle le biomarqueur est une séquence IL-8 ARNm ou ADN.

7. La sonde selon l'une quelconque des revendications précédentes, dans laquelle la séquence polynucléotidique est choisie dans le tableau 2-4.

8. Un procédé pour détecter un biomarqueur ARNm ou ADN dans un échantillon, le procédé comprenant :
- mettant en contact la sonde de l'une quelconque des revendications précédentes avec l'échantillon et le récepteur, et détectant la présence ou l'absence d'un complexe entre le ligand et le récepteur,
- dans lequel la présence du complexe indique la présence du biomarqueur ARNm ou ADN dans l'échantillon et l'absence du complexe indique l'absence du biomarqueur ARNm ou ADN dans l'échantillon.

9. Le procédé selon la revendication 8, dans lequel l'échantillon est un échantillon de salive.

10. Le procédé selon la revendication 8 ou 9, dans lequel le biomarqueur est une séquence IL-8 ARNm ou ADN.
